(19) Europäisches Patentamt / European Patent Office / Office européen des brevets

(11) EP 3 841 160 B1

(12) # FASCICULE DE BREVET EUROPEEN

(45) Date de publication et mention de la délivrance du brevet:
**11.05.2022 Bulletin 2022/19**

(21) Numéro de dépôt: **19752197.4**

(22) Date de dépôt: **13.08.2019**

(51) Classification Internationale des Brevets (IPC):
***C08J 11/18*** *(2006.01)* ***C07C 1/213*** *(2006.01)*
***C07C 1/22*** *(2006.01)* ***C07C 29/09*** *(2006.01)*
***C07C 31/20*** *(2006.01)* ***C07C 33/26*** *(2006.01)*
***C08K 5/54*** *(2006.01)*

(52) Classification Coopérative des Brevets (CPC):
(C-Sets disponibles)
**C08J 11/18; C07C 29/147; C07C 37/0555; C07C 41/26;** C08J 2367/00; C08J 2369/00; Y02W 30/62 (Cont.)

(86) Numéro de dépôt international:
**PCT/EP2019/071711**

(87) Numéro de publication internationale:
**WO 2020/038775 (27.02.2020 Gazette 2020/09)**

(54) **PROCEDE DE DEPOLYMERISATION DE MATERIAUX POLYMERES OXYGENES PAR CATALYSE NUCLEOPHILE**

VERFAHREN ZUR DEPOLYMERISATION VON OXYGENIERTEN POLYMERMATERIALIEN DURCH NUKLEOPHILE KATALYSE

METHOD FOR DEPOLYMERISING OXYGENATED POLYMER MATERIALS BY NUCLEOPHILIC CATALYSIS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.08.2018 FR 1857614**

(43) Date de publication de la demande:
**30.06.2021 Bulletin 2021/26**

(73) Titulaire: **Commissariat à l'Energie Atomique et aux Energies Alternatives**
**75015 Paris (FR)**

(72) Inventeurs:
- **MONSIGNY, Louis**
  **91440 Bures Sur Yvette (FR)**
- **CANTAT, Thibault**
  **92130 ISSY LES MOULINEAUX (FR)**

(74) Mandataire: **Gevers & Orès**
**Immeuble le Palatin 2**
**3 Cours du Triangle**
**CS 80165**
**92939 Paris La Défense Cedex (FR)**

(56) Documents cités:
**WO-A1-2016/098021**

- **ERIC M. KRALL ET AL: "Controlled hydrogenative depolymerization of polyesters and polycarbonates catalyzed by ruthenium(ii) PNN pincer complexes", CHEMICAL COMMUNICATIONS, vol. 50, no. 38, 1 janvier 2014 (2014-01-01), page 4884, XP055206893, ISSN: 1359-7345, DOI: 10.1039/c4cc00541d**
- **CÁTIA SANTOS NUNES ET AL: "PET depolymerisation in supercritical ethanol catalysed by [Bmim][BF4]", RSC ADVANCES, vol. 4, no. 39, 1 janvier 2014 (2014-01-01), page 20308, XP055210283, ISSN: 2046-2069, DOI: 10.1039/c4ra00262h**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 841 160 B1

(52) Classification Coopérative des Brevets (CPC):
(Cont.)

C-Sets
**C07C 29/147;**
**C07C 37/0555;**
**C07C 41/26**

## Description

La présente invention concerne un procédé de dépolymérisation de polymères oxygénés, notamment par catalyse nucléophile et l'utilisation dudit procédé dans le recyclage de matériaux plastiques et la préparation de composés aromatiques et aliphatiques pouvant être utilisés comme carburant, intermédiaires de synthèse, matières premières dans les secteurs de la construction, dans l'industrie pétrochimique, électrique, électronique, de textile, aéronautique, pharmaceutique, cosmétique, agrochimique.

La présente invention concerne également un procédé de fabrication de combustibles, de composants électroniques, de polymères plastiques, de caoutchouc, de médicaments, de vitamines, de produits cosmétiques, de parfums, de produits alimentaires, de fils et fibres synthétiques, de cuirs synthétiques, de colles, de pesticides, d'engrais comprenant (i) une étape de dépolymérisation de polymères oxygénés selon le procédé de l'invention et éventuellement (ii) une étape d'hydrolyse, et éventuellement (iii) une étape de fonctionnalisation et/ou défonctionnalisation.

Les polymères oxygénés sont actuellement des constituants de base d'une grande partie des matériaux de la vie courante et en particulier des plastiques. En effet, un matériau plastique est essentiellement constitué d'un polymère qui après des opérations de moulage et de façonnage, conduit à l'obtention d'un objet fini ou semi-fini. Ces plastiques ont généralement des masses moléculaires élevées et sont souvent issus de la pétrochimie, mais il existe des plastiques d'origine naturelle. De nos jours, un intérêt croissant envers les matériaux plastiques est observé en raison de leur facilité de fabrication, de leur coût relativement faible ainsi qu'à la polyvalence qu'ils présentent. Cependant, le coût relativement élevé du recyclage de ces matériaux en utilisant les procédés actuels pose des problèmes d'ordre économiques nécessitant de nouvelles solutions pouvant s'accorder avec l'évolution des législations. Ainsi, le recyclage des matériaux contenant des polymères oxygénés est devenu un enjeu important de la société contemporaine.

Plusieurs méthodes de recyclage ont été développées pour faire face à cette problématique. Parmi ces méthodes, le recyclage chimique (ou recyclage tertiaire) est une méthode de recyclage qui est en accord avec les principes du développement durable. En effet, ce type de recyclage permet de récupérer des constituants de la pétrochimie, des déchets des matériaux polymères et des déchets plastiques et de les utiliser comme précurseurs dans la création de produits à hautes valeurs ajoutées. Les matériaux polymères peuvent ainsi être considérés comme une source de matières carbonées (S.M. Al-Salem, P. Lettieri, J. Baeyens, Progress in Energy and Combustion Science, 2010, 36 (2010) pages 103-129 ; S. H. Park and S. H. Kim, Fashion and Textiles, 2014, 1, pages 1-17).

Les méthodes de recyclage chimiques sont généralement divisées en deux catégories : celles qui régénèrent les monomères ou des oligomères de départ (réactions d'hydrolyse) et celles qui génèrent d'autres types de molécules ayant des applications en chimie fine ou comme carburant (réactions de transestérifications, d'aminolyse, de méthanolyse, de glycolyse, etc.). De nombreuses méthodes chimiques de recyclage existent dans la littérature (D. S. Achilias, D. A. Louka, G. Tsintzou, I. A. Koutsidis, I. Tsagkalias, L. Andriotis, N. P. Nianias, P. Siafaka, Recent Advances in the Chemical Recycling of Polymers (PP, PS, LDPE, HDPE, PVC, PC, Nylon, PMMA) ; INTECH Open Access Publisher, 2012, ISBN: 953510327X, 9789535103271 ; Chemically recyclable polymers: a circular economy approach to sustainability, Miao Hong and Eugene Y.-X. Chen, Green Chem., 2017, 19, 3692) etc.

Récemment, deux méthodes de choix ont été développées : la réduction des composés oxygénés avec l'hydrogène moléculaire et les hydrosilanes.

### A) Hydrogénation

L'hydrogénation est une méthode initialement développée par Robertson et al. (Chem. Commun., 2014, 50, 4884-4887) qui permet le recyclage de plusieurs types de polymères comme les polyesters et les polycarbonates. Cette méthode utilise un complexe de ruthénium PNN à 1 % mol, silane

. Les réactions se réalisent en présence d'anisole comme co-solvant afin d'augmenter la solubilité des polymères, et à une température de 160°C et une pression de 54,4 atm de $H_2$. Le PLA et le PET ainsi que quelques polycarbonates (le PC-BPA n'était pas testé) ont été dépolymérisés pour fournir pour la première fois des diols avec des conversions allant de 91 à >99 % après 48 h de réaction. Récemment, Klankermayer *et al.* ont developpé un catalyseur à base de ruthenium Ru(triphos)TMM et de l'acide triflimidique comme cocatalyseur (S. Westhues, J. Idel, J. Klankermayer, Molecular catalyst systems as key enablers for tailored polyesters and polycarbonate recycling concepts. Sci. Adv., 2018, 4, eaat9669). Ce nouveau système réalise la réduction de polyesters et de polycarbonates à 100 bar d'hydrogène et 120 °C et requiert une quantité de 0.1 à 1 mol% de catalyseur Ru(triphos)TMM et de l'acide triflimidique. Comme le système de Robertson, le système de Klankermayer permet de récupérer des diols à partir de PET, de PLA mais aussi de PC-BPA avec des rendements isolés allant de 73 à 93%. Ces systèmes tolèrent les impuretés et les additifs présents dans les bouteilles de PET et de PLA. Cependant, il présente les désavantages suivants :

1. Les réactions de dépolymérisations se réalisent à des températures et des pressions élevées (*i.e.* températures >100°C et pressions entre 10 et 100 bars).

2. Les réactions nécessitent l'ajout d'additifs comme l'anisole (comme co-solvant) et le tBuOK (sensible à l'air) ou encore l'acide triflimidique pour activer les catalyseurs lesquels catalyseurs promouvront la réaction d'hydrogénation. L'ajout de ces additifs généralement issus d'une source non renouvelable implique l'utilisation d'une grande quantité de réactifs carbonés de départ qui nécessiteront, par la suite, une étape additionnelle de recyclage pour les récupérer.
3. L'utilisation de complexes contenant un métal noble (le ruthénium) et qui sont non commerciaux. En effet, la synthèse des complexes doit s'effectuer en plusieurs étapes et nécessite un matériel sophistiqué avec un coût élevé.

**B) Hydrosilylation (par activation électrophile de l'hydrosilane)**

Cette méthode a été récemment développée par le groupe de Cantat (Chem Sus Chem, 2015, 8, 980 - 984 ; WO2016/098021, ACS Sustainable Chem. Eng., 2018, 6 , 10481-10488) permettant le recyclage de polyéthers, polyesters et polycarbonates. Cette méthode fait appel à un catalyseur de type acide de Lewis. La réaction est effectuée selon un mécanisme d'activation électrophile de l'hydrosilane ; un intermédiaire silylé cationique est généré à l'aide d'un promoteur acide de Lewis pour réaliser la dépolymérisation de plastiques tels que le PLA, le PET et le PC-PBA en éthers silylés ou en alcanes avec des conversions allant de 30 à 99% comme montré en figure 1. Cette réaction repose entièrement sur l'utilisation du promoteur (idéalement un catalyseur) de type acide de Lewis. Ce système tolère les impuretés et les additifs présents dans les bouteilles de PET. En plus des liaisons esters et carbonates, ce système s'est montré efficace dans le clivage des liaisons éthers. Cependant, des améliorations peuvent y être apportées, en particulier pour

- améliorer la sélectivité du système, notamment vis-vis du clivage sélectif de certaines liaisons du polymère oxygéné. Par exemple, si le polymère oxygéné contient des liaisons esters et des liaisons éthers comme la polydioxanone (PDO), cliver sélectivement les liaisons esters en laissant les liaisons éthers intacte,
- recourir aux solvants moins dangereux pour l'homme et pour l' environnement,
- Effectuer la mise en œuvre de la dépolymérisation des plastiques oxygénés dans des conditions plus intéressantes du point de vue industrielle, comme par exemple, l'utilisation de catalyseurs au moins aussi efficaces mais moins onéreux et moins sensibles à l'air et/ou à l'eau.

Les acides de Lewis ont montré leur efficacité dans la réduction des biopolymères très difficiles à dépolymériser comme par exemple la méthylcellulose (Gagné et al. Metal-Free Deoxygenation of Carbohydrates ; Angew. Chem. Int. Ed. 2014, 53, 1646 -1649 Gagné et al. (Metal-Free Deoxygenation of Carbohydrates ; Angew. Chem. Int. Ed. 2014, 53, 1646 -1649) ou la lignine (Feghali et al. Energy Environ. Sci., 2015, 8, 2734-2743 et Monsigny et al., Green Chem., 2018, 20, 1981-1986). Dans ces systèmes, le clivage intervient au niveau des liaisons carbone sp3 - oxygène (éthers et alcools) comme montré en figure 2.

**C) Hydrosilylation (par activation nucléophile de l'hydrosilane)**

La réactivité chimique ainsi que les applications de catalyseurs de type base de Lewis (alkoxide de metal et/ou source de fluorure, etc.) en hydrosilylation ont été examinés en détail dans la littérature (K. Revunova and G. I. Nikonov, Dalton Trans., 2014, DOI: 10.1039/C4DT02024C).

En particulier, la réaction d'hydrosilylation en utilisant ce type de catalyseur et/ou initiateur est très importante en ce sens qu'elle ouvre de nouvelles voies de synthèse pour obtenir de nouveaux composés en utilisant des conditions douces. De plus, les bases de Lewis utilisées sont généralement moins coûteuses que les catalyseurs permettant l'activation électrophile des hydrosilanes (Angew. Chem. Int. Ed. 2015, 54, 6931 -6934). La réaction d'hydrosilylation catalysée par du fluorure de césium CsF a initialement été étudiée par Volpin *et al.*, par Corriu *et al.* et par Hiyama *et al.* (M. Deneux, I.C. Akhrem, D.V. Avetissian, E.I. Mysoff and M.E. Volpin, Bull. Soc. Chim. France, (1973) 2638 ; Boyer, J.; Corriu, R. J. P.; Perz, R.; Reye, C. J. Organomet. Chem. 1979, 172, 143 ; Corriu, R. J. P.; Perz, R.; Reye, C. Tetrahedron 1983, 39, 999 ; M. Fujita and T. Hiyama, J. Am. Chem. Soc., 106 (1984) 4629.). Cette réaction a directement suscité un intérêt considérable de plusieurs groupes de recherche dans le monde. L'utilisation de nucléophile comme un catalyseur dans la réaction d'hydrosilylation a permis la réduction d'une grande variété de substrats organiques comme les aldéhydes, les imines et les acides carboxyliques, les esters et les amides. Cependant, la réaction n'a jamais été développée sur les carbonates à cause de la faible réactivité de ce type de fonctions chimiques et par conséquent, leur difficulté a être réduites. (Dub, P. A.; Ikariya, T. Catalytic Reductive Transformations of Carboxylic and Carbonic Acid Derivatives Using Molecular Hydrogen. ACS Catal. 2012, 2 (8), 1718-1741).

Par ailleurs, bien que la réaction d'hydrosilylation par activation nucléophile présente des avantages, elle n'a jamais été utilisée dans une réaction de dépolymérisation. Ceci peut s'expliquer de plusieurs manières :

- La réaction a été développée initialement pour des applications en chimie de synthèse et ne visait pas la valorisation

dans le domaine des matériaux qui sont souvent insolubles dans les solvants classiques de chimie moléculaire.

- Les hydrosilanes classiquement utilisés dans les réductions, notamment ceux qui ne sont pas des sous-produits de l'industrie des silicones, sont coûteux.
- Les matériaux polymériques et les déchets plastiques en particulier, contiennent une grande quantité de contaminants et/ou additifs (tels que les pigments, etc), ce qui peut désactiver la plupart des catalyseurs.
- Les conditions opératoires mises au point sur des petites molécules ou des molécules modèles, ne sont pas nécessairement transposables aux polymères comprenant les mêmes fonctionnalités. En effet, alors que les petites molécules sont souvent solubles dans les solvants classiques de chimie organique, les polymères correspondants ne le sont pas forcément ce qui peut altérer de façon importante la réactivité de ces polymères.

Il faut toutefois signaler que Grubbs et al. (Chem. Sci., 2013,4, 1640-1645) ont étudié l'utilisation de terbutanolate de potassium (KOtBu) de manière stœchiométrique et non catalytique (2-3 équivalents) en présence de triéthylsilane ($Et_3SiH$) pour la réduction de liaisons aryl éther dans des molécules modèles de lignine, c'est-à-dire, de liaison carbone oxygène entre deux aromatiques dans une molécule mimant une liaison présente dans un polymère de lignine (figure 3). Cette réaction a mené à un mélange complexe de produits et, plus particulièrement, à une réaction de silylation des carbones aromatiques (appelée C-silylation) via un mécanisme radicalaire.

Toutefois, les méthodes de recyclage tertiaire présentent des inconvénients opérationnels non négligeables comme la conduite de la réaction à haute température et à hautes pressions ainsi que l'utilisation des métaux et/ou de composés onéreux pour catalyser les réactions. De plus, rares sont les méthodes permettant de recycler à la fois plusieurs types de polymères (recyclage de copolymères ou de mélange de polymères) et qui résistent aux additifs et/ou impuretés présents dans les polymères.

Ainsi, il existe un réel besoin de développer une méthode alternative aux méthodes de recyclage tertiaire des polymères déjà existantes, en particulier des polymères oxygénés, en composés ayant une haute valeur ajoutée palliant les inconvénients des méthodes de recyclage tertiaire de l'art antérieur.

En particulier, il existe donc un réel besoin de développer un procédé de dépolymérisation pouvant être appliqué au recyclage des polymères, en particulier des polymères oxygénés, en composés ayant une haute valeur ajoutée.

Plus particulièrement, il existe un réel besoin d'un procédé de dépolymérisation de polymères, en particulier les polymères oxygénés :

- qui conduise à la formation de composés moins oxygénés (présentant moins d'oxygène dans la formule brute du composé ou encore moins de liaisons C-O, ou en d'autres termes des composés ayant un ratio d'atomes O/(C+H) moins élevé que le composé de départ ;
- respectueux de l'environnement ;
- pouvant être mis en œuvre dans des conditions opératoires douces et industriellement intéressantes ;
- évitant l'utilisation de catalyseurs à base de métaux polluants et coûteux ;
- qui soit efficace, l'efficacité se traduisant par une bonne conversion (au moins 50% molaire par rapport au nombre de motifs monomères du polymère de départ) voire une conversion totale du polymère oxygéné en composés chimiques,
- avec une bonne pureté (au moins 90 % molaire par rapport au nombre total de mole de composés obtenus) ou pouvant être facilement purifiés ;
- avec une bonne sélectivité par rapport aux composés chimiques obtenus ;
- permettant un clivage sélectif de certaines liaisons du polymère oxygéné ;
- qui soit général et versatile pouvant s'adapter au polymère oxygéné à dépolymériser ; et/ou
- capable de résister aux additifs et/ou impuretés (ou contaminants) éventuellement présents dans les polymères oxygénés à dépolymériser.

La présente invention a précisément pour but de répondre à ces besoins en fournissant un procédé de dépolymérisation de polymères oxygénés par clivage sélectif des liaisons oxygène-carbone des fonctions esters (-CO-O-) et des fonctions carbonates (-O-CO-O-), caractérisé en ce qu'il comprend une étape de mise en contact desdits polymères oxygénés avec un composé hydrosilane de formule (I)

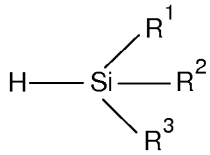

(I)

dans laquelle

- $R^1$, $R^2$ et $R^3$ représentent, indépendamment l'un de l'autre, un atome d'hydrogène, un atome d'halogène, un groupe hydroxyle, un groupe alkyle, un groupe alcényle, un groupe alcynyle, un groupe alkoxy, un groupe aryloxy, un groupe silylé, un groupe siloxy, un groupe aryle, un groupe amino, lesdits groupes alkyle, alcényle, alcynyle, alkoxy, silylé, siloxy, aryle et amino étant éventuellement substitués, ou
- $R^1$ est tel que défini ci-dessus et $R^2$ et $R^3$, pris ensemble avec l'atome de silicium auquel ils sont liés forment un hétérocycle silylé éventuellement substitué ;

en présence d'un catalyseur de type base de Lewis.

Plus particulièrement, l'invention a pour objet un procédé de dépolymérisation de polymères oxygénés par clivage sélectif des liaisons oxygène-carbone des fonctions esters (-CO-O-) et des fonctions carbonates (-O-CO-O-), caractérisé en ce qu'il comprend une étape de mise en contact desdits polymères oxygénés avec un composé hydrosilane de formule (I)

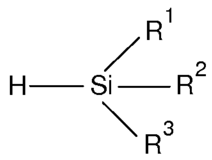

(I)

dans laquelle

- $R^1$, $R^2$ et $R^3$ représentent, indépendamment l'un de l'autre, un atome d'hydrogène, un atome d'halogène, un groupe hydroxyle, un groupe alkyle, un groupe alcényle, un groupe alcynyle, un groupe alkoxy, un groupe aryloxy, un groupe silylé, un groupe siloxy, un groupe aryle, un groupe amino, lesdits groupes alkyle, alcényle, alcynyle, alkoxy, silylé, siloxy, aryle et amino étant éventuellement substitués, ou
- $R^1$ est tel que défini ci-dessus et $R^2$ et $R^3$, pris ensemble avec l'atome de silicium auquel ils sont liés forment un hétérocycle silylé éventuellement substitué ;

en présence d'un catalyseur de type base de Lewis, ledit catalyseur de type base de Lewis étant

- un alcoolate de formule (II)

  $(R^6\text{-O-})_w\ M^{w+}$ (II)

  dans laquelle

  - w est 1, 2, 3, 4, et 5 ;
  - $R^6$ est un alkyle comprenant 1 à 6 atomes de carbone, un alcényle comprenant 2 à 6 atomes de carbone, un alcynyle comprenant 2 à 6 atomes de carbone ou un aryle monocyclique ou polycyclique typiquement bi- ou tri- cyclique comprenant 6 à 20 atomes de carbone ; et
  - M est un métal choisi parmi Li, Na, K, Cs ou Rb Cu, Mg, Zn, Ca, Sr, Ba, Pb, Al, Sb, La,. Zr, Si, Ti, Sn, Hf, Ge, V ; ou

- un composé permettant de libérer un fluorure ($F^-$) de formule (III) :

  $Y^{z+}\text{ - }(F^-)_z$ (III)

  dans laquelle

  - z est 1, 2, 3, 4;
  - Y est un ammonium d'alkyle dont l'alkyle comprend 1 à 6 atomes de carbone, un ammonium d'alcényle dont l'alcényle comprend 2 à 6 atomes de carbone, un ammonium d'alcynyle dont l'alcynyle comprend 2 à 6 atomes de carbone ou un aryle comprenant 6 à 10 atomes de carbone ; un ammonium de quinine, ou Y est un métal choisi parmi Li, Na, K, Cs, Rb, Cu, Zn, Ca, Ba, Al, Zr, Sn ;

- un fluorosilicate choisi parmi :

- les hexafluorosilicates $SiF_6^{2-}$ avec un contre-ion alcalin choisi parmi Li, Na, K et Cs ; ou
- les fluorosilicates de formule $(R^7)_3SiF_2^-$ avec un contre-ion ammonium d'alkyle de formule $N(R^{10})_4^+$ou un contre-ion sulfonium de formule $S(R^{11})_3^+$; avec

  - $R^7$ étant un alkyle comprenant de 1 à 6 atomes de carbone choisi parmi le méthyle, l'éthyle, le propyle, le butyle, le pentyle ou le hexyle et leurs isomères ramifiés ; ou un aryle comprenant de 6 à 10 atomes de carbone choisi parmi le phényle, le benzyle ou le naphtyle ;

  - $R^{10}$ étant un atome d'hydrogène ; un méthyle, un éthyle, un propyle, un butyle, et leurs isomères ramifiés.

  - $R^{11}$ étant un atome d'hydrogène ; un méthyle, un éthyle, un propyle, un butyle, et leurs isomères ramifiés ou des amines primaires, secondaires ou tertiaires ;

- un amidure primaire ou secondaire un dérivé guanidine choisi parmi

  - les guanidinates bicycliques de sodium ou potassium, notamment le sel de sodium ou de potassium de l'anion du 1,3,4,6,7,8-hexahydro-2H-pyrimido[1,2-a]pyrimidine (ou Hhpp),
  - la guanidine,
  - la 1,5,7-triazabicyclo[4.4.0]dec-5-ène (ou TBD) ;

- un hétérocycle carbènique de formule générale (IV) :

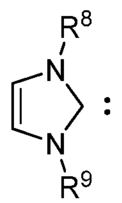

(IV)

dans laquelle

  - $R^8$ et $R^9$ représentent, indépendamment l'un de l'autre, un alkyle comprenant 1 à 6 atomes de carbone, un alcényle comprenant 2 à 6 atomes de carbone, un alcynyle comprenant 2 à 6 atomes de carbone ou un aryle bi- ou tri- cyclique comprenant 6 à 20 atomes de carbone ;

- un carbonate de formule (V)

$$M'_2CO_3 \qquad (V)$$

dans laquelle M' est un métal choisi parmi Li, Na, K, Cs ou Rb.

Au sens de l'invention, un polymère oxygéné est un polymère comprenant au moins un polymère oxygéné et éventuellement au moins un additif et/ou une impureté (ou contaminant).

Dans le contexte de la présente invention, un polymère oxygéné désigne un polymère ou copolymère dont les motifs de répétition de la chaîne principale contiennent la fonction ester (-CO-O-) appelés également les polyesters ou la fonction carbonate (-O-CO-O-) appelés également les polycarbonates.

Les polymères oxygénés de l'invention sont principalement des polymères synthétiques ou semi-synthétiques mais peuvent aussi être des polymères naturels et biosourcés, c'est-à-dire, issus de la biomasse animale ou végétale. Le ou les additifs éventuellement présents dans le matériau peuvent être introduits avant ou pendant la mise en forme du matériau, pour apporter ou améliorer une (ou parfois plusieurs) propriété(s) spécifique(s). A titre d'exemple d'additifs on peut citer les stabilisants, les antioxydants, les colorants, les pigments, les agents mouillants, les dispersants, les émulsifiants, les épaississants, les biocides, les plastifiants, les photoprotecteurs, etc.

Dans le contexte de la présente invention, les termes « impuretés » et « contaminants » désignent des composés ayant été en contact avec le polymère selon son origine et son usage (par exemple des protéines dans le cas d'une bouteille de lait, du sucre dans les bouteilles de soda, de la colle utilisée pour coller les étiquettes sur les bouteilles, etc.). Dans cet exposé, les termes impuretés et contaminants peuvent être utilisés indifféremment.

Le procédé de l'invention a l'avantage de résister à la présence d'additif(s) et/ou impuretés (ou contaminants)

dans les polymères. Aucun problème d'empoisonnement de catalyseur n'a pu être observé avec les additifs couramment utilisés dans les polymères. En effet, le plus grand défi du recyclage ne se limite pas à la dépolymérisation du polymère présent seul dans le milieu réactionnel (polymère pur) mais s'étend également à sa dépolymérisation dans un matériau commercial pouvant contenir des additifs comme des colorants, des charges minérales, des antioxydants, etc. La présence de ces additifs dans le matériau peut désactiver le catalyseur employé pour réaliser la dépolymérisation, et ainsi rendre la réaction inefficace. Le procédé de l'invention présente donc un grand intérêt industriel car il est capable de résister aux additifs et/ou impuretés (ou contaminants) présents dans le polymère de départ, qui, par exemple, peut être un déchet plastique.

Les bases de Lewis utilisées dans cette invention comme catalyseur ont l'avantage d'être généralement moins coûteuses que les acides de Lewis et peuvent être employées en l'absence de solvants ou dans des solvants généralement peu nocifs tels que le THF, le méthylTHF, l'anisole, PEG 400, etc.

La réaction d'hydrosilylation catalysée par des bases de Lewis est plus sélective que la réaction d'hydrosilylation catalysée par des acides de Lewis puisque les liaisons carbone $sp^3$-oxygène restent intactes.

Le procédé de dépolymérisation de l'invention conduit à la formation d'un seul produit sous forme d'éther de silyle, c'est-à-dire un composé chimique contenant des groupes siloxy ($-O-SiR_1R_2R_3$). L'hydrolyse dudit éther de silyle peut avoir lieu *in situ* sans étape de traitements intermédiaires *i.e* sans isoler les produits siloxy.

Les hydrosilanes employés dans ce nouveau procédé sont bon marché et produits à grande échelle.

Les polymères oxygénés de départ peuvent être un polymère oxygéné, ou un mélange de polymères oxygénés, ou un mélange d'au moins deux polymères dont l'un au moins est un polymère oxygéné au sens de l'invention, avec éventuellement un ou plusieurs additif(s) et/ou une impureté (ou contaminant).

Selon un mode de réalisation de l'invention, les matériaux oxygénés de l'invention comprennent un ou plusieurs additif(s).

Lorsque le polymère oxygéné est un copolymère, la chaîne principale dudit copolymère peut comporter des motifs de répétition contenant une ou plusieurs fonctions ester (-CO-O-) et/ou une ou plusieurs fonctions carbonate (-O-CO-O-) et éventuellement des motifs de répétition choisis parmi des motifs éthylénique, propylénique, vinylique, vinylique substitué par un ou plusieurs atomes de chlore ou de fluor, styrénique, styrène-butadiène, acrylique, méthacrylique.

Dans la suite de l'exposé, le terme « polymère » peut également désigner un « copolymère ». Ainsi, le terme polymère peut couvrir les homopolymères (un polymère issu d'une seule espèce de monomère) et les copolymères (un polymère issu d'au moins deux monomères différents).

Les polymères oxygénés synthétiques ou semi-synthétiques de l'invention peuvent être choisis parmi :

- les polyesters saturés ou insaturés choisis, par exemple, parmi l'acide polylactique (PLA), le polycaprolactone (PCL), le polyhydroxyalkanoate (PHA), le polyhydroxyvalérate (PHV), le polyéthylène adipate (PEA), le polybutylène succinate (PBS), le poly(3-hydroxybutyrate-co-3-hydroxyvalérate (PHBV), le polyéthylène téréphthalate (PET), le polybutylène téréphthalate (PBT), le polytriméthylène téréphthalate (PTT), le polyéthylène naphthalate (PEN), la polydioxanone (PDO) ;
- les polycarbonates choisis, par exemple, parmi le PC-BPA, le polypropylène carbonate (PPC), le polyéthylène carbonate (PEC), le poly(hexaméthylène carbonate), l'allyl diglycol carbonate (ADC) ou CR-39.

Parmi les polymères oxygénés, le polyéthylène téréphtalate (PET), l'acide polylactique (PLA) et le polycarbonate (PC-BPA) sont les plus étudiés dans la littérature car leur recyclage présente plusieurs intérêts :

➢ Le polytéréphtalate d'éthylène (PET) est l'un des plastiques les plus couramment utilisés dans le monde du fait de sa légèreté, de sa durabilité, de sa résistance chimique mais aussi de son faible coût.

➢ L'acide polylactique (PLA) est très intéressant d'un point de vue environnemental puisqu'il est principalement dérivé de ressources renouvelables, telles que le maïs, les pommes de terre et d'autres produits agricoles. En raison de sa biodegrabilité combinée à sa résistance mécanique et sa transparence, le PLA est considéré comme un matériau vert et durable qui peut être vu comme une alternative prometteuse aux résines polymères à base de pétrole notamment le PET.

➢ Les polycarbonates (PC-BPA) sont des thermoplastiques ayant d'excellentes propriétés mécaniques et une grande résistance aux chocs, une résistance aux UV, ainsi qu'une excellente résistance électrique. En conséquence, les polycarbonates sont utilisés dans une grande variété d'applications comme dans les disques compacts, les fenêtres blindées, les emballages alimentaires ou les bouteilles de boissons gazeuses.

Dans le cadre de l'invention, les polymères oxygénés peuvent également être biosourcés et être, plus particulièrement, issus de la biomasse végétale dont les motifs aromatiques sont liés par des liaisons esters. A ce titre, on peut citer les tannins hydrolysables notamment les gallotannins et les ellagitannins, et la subérine.

Les polymères oxygénés sont avantageusement choisis parmi

- les polyesters choisis parmi le PET, le PCL, le PDO ou le PLA ;
- les polycarbonates choisis parmi le PC-BPA ou le PPC.
- les tannins hydrolysables choisis parmi les gallotannins, les ellagitannins, ou la subérine.

Comme déjà indiqué, les matériaux de l'invention peuvent être un mélange d'au moins deux polymères dont l'un au moins est un polymère oxygéné au sens de l'invention. Dans ce cas, le ou les autres polymère(s) présent(s) dans le matériau peu(ven)t être choisi(s) parmi les polyoléfines notamment le polyéthylène et le polypropylène ; le polyacétate de vinyle (PVAC), l'alcool polyvinylique (PVAL) ; le polystyrène (PS), l'acrylonitrile butadiène styrène (ABS) ; le styrène-butadiène (SBR) ; l'acrylonitrile styrène acrylate (ASA) ; les polyuréthanes saturés ou réticulés ; le polyméthacrylate de méthyle (PMMA), le polyacrylonitrile (PAN) ; le polychlorure de vinyle (PVC), le polychlorure de vinylidène (PVDC) ; le polytétrafluoroéthylène (PTFE), le polyfluorure de vinyle (PVF), le polyfluorure de vinylidène (PVDF), l'éthylène tétrafluoroéthylène (ETFE), le perfluoroalkoxy (PFA) ; le polyétheréthercétone (PEEK) ; les copolymères séquencés styrène-butadiène-styrène (SBS) ; les polyamides comme le PA6, le PA 12, le PA 6.6 ; les polyuréthanes ; les polyurées ; les copolymères de polyurées et de polyuréthanes (connus sous les nom de Spandex, Lycra ou élastane).

En contrôlant minutieusement les conditions opératoires, la dépolymérisation d'un polymère comprenant un mélange de polyester(s) et/ou de polycarbonate(s) peut être sélective. Par exemple, dans le cas d'un mélange PPC+PLA, le PLA est clivé en premier. Dans le cas d'un mélange de PC-BPA + PLA, c'est la PC-PBA qui est clivé en premier à cause des effets électronique. Dans le cas d'un mélange PET + PLA, le PET est clivé en premier en raison des effets électroniques.

Le procédé de dépolymérisation selon l'invention peut conduire en général à la formation de composés chimiques à l'état liquide comme l'éthylène glycol et/ou à l'état solide comme le bisphénol A (BPA).

Quelle que soit la nature du polymère de départ utilisé, la nature de l'hydrosilane et sa quantité, le catalyseur employé et la durée de la réaction, la nature du produit de dépolymérisation reste inchangé. Ainsi, les produits obtenus sont toujours des éthers de silyle qui pourront être hydrolysés pour fournir les diols correspondant.

En effet, l'un des avantages du procédé de l'invention est la grande sélectivité vers un seul type de produit obtenu à partir d'un même polymère. Par exemple, dans le cas du PET, le procédé de dépolymérisation conduit uniquement à l'obtention du 1,4-phénylène diméthanol et de l'éthylène glycol. Aucun autre produit n'est observé dans la dépolymérisation du PET.

Dans le cadre de la présente invention, la sélectivité se rapporte à la nature des produits formés ainsi qu'à la nature des liaisons clivées.

Les liaisons visées et clivées sélectivement par le procédé de dépolymérisation de l'invention sont les liaisons oxygène-carbone de type carbonyles des fonctions esters (-CO-O-) et des fonctions carbonates (-O-CO-O). Ainsi, les liaisons C-O des fonctionnalités dans lesquelles l'atome de carbone est lié à un autre atome de carbone par une liaison simple carbone $sp^3$ - oxygène, une liaison multiple sp ou $sp^2$ (par exemple C=C-O) ne sont pas clivées lors du procédé de dépolymérisation de l'invention. Par exemple, les alkyles éthers présents dans le polyéthylène glycol (PEG) ou encore les aryles éthers présents dans les polyphénols, ne sont pas clivés. Les liaisons C-C simples, doubles et triples ne sont pas non plus clivées par le procédé de dépolymérisation de l'invention. Par exemple, le polystyrène (PS) n'est pas dépolymérisé par le procédé de l'invention.

Selon les conditions opératoires, pendant le procédé de dépolymérisation, la fonction carbonyle -C=O est réduite en un éther silylé -CH-OSi$R^1R^2R^3$ où $R^1$, $R^2$, et $R^3$ sont tels que définis pour la formule (I) dans le cadre de la présente invention.

Le procédé de dépolymérisation de l'invention est d'une grande versatilité notamment par rapport aux types de matériaux polymères oxygénés de départ.

D'autre part, l'étape de dépolymérisation dans le procédé de l'invention peut être réalisé dans des conditions opératoires douces, *i.e.* températures douces (15°C à 75°C) et basses pressions (1 à 5 bars, de préférence 1 à 2 bars) et permet de s'affranchir des conditions réactionnelles drastiques de température et de pression utilisées traditionnellement, par exemple, dans le recyclage des matériaux polymères.

De plus, l'utilisation de solvants dits « verts » est un atout pour ce système. Dans certaines conditions de la présente invention, aucun solvant n'est utilisé. Ceci est un avantage important pour le respect de l'environnement.

Le procédé de dépolymérisation des matériaux polymères oxygénés selon l'invention fournit des composés chimiques pouvant contenir des groupes siloxy et ayant un nombre de carbones plus réduit que celui du ou des polymères oxygénés présent(s) dans le matériau de départ. Les composés obtenus peuvent conduire, après réactions d'hydrolyse, à des composés chimiques de masse molaire moyenne inférieure à 600 g/mol.

Par ailleurs, le rendement en composés chimiques de masse molaire moyenne inférieure à 600 g/mol obtenus par le procédé de dépolymérisation et après l'étape d'hydrolyse, dépend du matériau polymère de départ ainsi que des conditions opératoires appliquées. Le rendement est en général bon (de 68 à 98 % molaire par rapport au nombre total de moles de motifs monomères présents dans le(s) polymère(s) du matériau de départ). Par approximation, et afin de calculer le rendement molaire du procédé de dépolymérisation, le matériau polymère de départ est considéré être

exclusivement formé du polymère étudié.

Le rendement est alors calculé en appliquant la formule suivante :

$$\text{Rendement} = \text{n (molécule cible) / n (motifs monomères) x 100}$$

avec

n (molécule cible) étant le nombre de moles de la molécule que l'on cherche à obtenir après dépolymérisation et ayant une masse molaire moyenne inférieure à 600 g/mol obtenu après hydrolyse, et
n (motifs monomères) étant le nombre de moles totales de motifs monomères présents dans les polymères de départ.

La pureté des molécules obtenues après dépolymérisation peut être calculée de la manière suivante :

$$\text{Pureté} = \text{n (molécule cible) / n (molécules obtenues issus du polymère) x 100}$$

avec

n (molécule cible) étant le nombre de moles de la molécule que l'on cherche à obtenir après dépolymérisation et ayant une masse molaire moyenne inférieure à 600 g/mol obtenu après hydrolyse, et
n (molécules obtenues issus du polymère) étant le nombre de moles de toutes les molécules dérivés des motifs monomères du polymère, y compris les sous-produits, obtenus après la réaction de dépolymérisation.

La conversion du polymère oxygéné de départ après dépolymérisation peut être calculée de la manière suivante :

$$\text{Conversion} = \text{n (molécules obtenues) / n (motifs monomères) x 100}$$

avec
n (molécules obtenues) et n (motifs monomères) tels que définis ci-dessus.

La dépolymérisation de polymères oxygénés naturels, biosourcés, synthétiques ou semisynthétiques peut générer des molécules aromatiques mono-, bi- et/ou tri-cycliques et mono- ou polyoxygénées comme, par exemple, di- et/ou tri-oxygénés. Par exemple, dans le cas de la dépolymérisation de polymères oxygénés naturels, les produits de dépolymérisation peuvent être des composés aromatiques monocycliques (cas de la gallotannin, par exemple), mono- bi- et/ou tri-cycliques (cas de l'ellagitannin, par exemple) et éventuellement mono-, di-, et/ou tri-oxygénés.

La dépolymérisation de polymères oxygénés naturels, bisourcés, synthétiques ou semi-synthétiques peut également générer des molécules non aromatiques (ou aliphatiques), saturées ou insaturées, présentant des liaisons éthers (ou non), constitués d'atomes de carbone et d'hydrogène pouvant être mono-, di-, et/ou tri-oxygénées.

On entend par « alkyle », au sens de la présente invention, un radical carboné linéaire, ramifié ou cyclique, saturé, éventuellement substitué, comprenant 1 à 12 atomes de carbone, de préférence 1 à 8 atomes de carbone, par exemple de 1 à 6 atomes de carbone. A titre d'alkyle saturé, linéaire ou ramifié, on peut citer par exemple les radicaux méthyle, éthyle, propyle, butyle, pentyle, hexyle, octyle, nonyle, décyle, undécyle, dodécanyle et leurs isomères ramifiés. Comme alkyle cylique, on peut citer les radicaux cyclopropyle, cyclobutyle, cyclopentyle, cyclohexyle, bicylco[2,1,1] hexyle, bicyclo[2,2,1] heptyle. Comme alkyles cycliques portant une insaturation, on peut citer par exemple le cyclopentényle, le cyclohexényle.

Par « alcényle » ou « alcynyle », on entend un radical carboné insaturé linéaire, ramifié ou cyclique, éventuellement substitué, ledit radical carboné insaturé comprenant 2 à 12 atomes de carbone, de préférence 2 à 8 atomes de carbone, par exemple 2 à 6 atomes de carbone, comprenant au moins une double liaison (alcényle) ou une triple liaison (alcynyle). A ce titre, on peut citer, par exemple, les radicaux éthylényle, propylényle, butényle, pentényle, hexényle, acétylényle, propynyle, butynyle, pentynyle, hexynyle et leurs isomères ramifiés.

Lorsque dans les radicaux alkyle, alcényle et alcynyle tels que définis ci-dessus au moins 1 atome de carbone $sp^3$, est remplacé par au moins un hétéroatome choisis parmi l'azote, l'oxygène, le bore, le silicium, le phosphore ou le soufre, il s'agit d'un radical « hétéroalkyle », « hétéroalcényle » et « hétéroalcynyle », respectivement. A titre indicatif, on peut citer le méthoxyle, l'éthoxyle, le butoxyle, le pentoxyle, le thiomethoxyle, le dimethylaminyle et leurs isomères ramifiés.

Les groupes alkyle, hétéroalkyle, alcényle, hétéroalcényle, alcynyle, et hélétoalcynyle au sens de l'invention,

peuvent être éventuellement substitués par un ou plusieurs groupes hydroxyle ; un ou plusieurs groupes alkoxy ; un ou plusieurs groupes siloxy ; un ou plusieurs atomes d'halogène choisis parmi les atomes de fluor, chlore, brome et iode; un ou plusieurs groupes nitro ($-NO_2$) ; un ou plusieurs groupes nitrile (-CN) ; un ou plusieurs groupes aryle ; avec les groupes alkoxy, siloxy et aryle tels que définis dans le cadre de la présente invention.

Le terme « aryle » désigne de manière générale un substituant aromatique cyclique comportant de 6 à 20, de préférence de 6 à 12 atomes de carbone, par exemple 6 à 10 atomes de carbone. Dans le cadre de l'invention le groupe aryle peut être mono- ou polycyclique. A titre indicatif, on peut citer les groupes phényle, benzyle et naphtyle. Le groupe aryle peut être éventuellement substitué par un ou plusieurs groupes hydroxyle, un ou plusieurs groupes alkoxy, un ou plusieurs groupes siloxy, un ou plusieurs atomes d'halogène choisis parmi les atomes de fluor, chlore, brome et iode, un ou plusieurs groupes nitro ($-NO_2$), un ou plusieurs groupes nitrile (-CN), un ou plusieurs groupes alkyles, avec les groupes alkoxy, siloxy et alkyle tels que définis dans le cadre de la présente invention.

Le terme « hétéroaryle » désigne de manière générale un substituant aromatique mono- ou polycyclique comportant de 5 à 10 membres, de préférence de 5 à 7 membres dont au moins 2 atomes de carbone, et au moins un hétéroatome choisi parmi l'azote, l'oxygène, le bore, le silicium, le phosphore et le soufre. Le groupe hétéroaryle peut être mono- ou polycyclique. A titre indicatif, on peut citer les groupes furyle, benzofuranyle, pyrrolyle, indolyle, isoindolyle, azaindolyle, thiophényle, benzothiophényle, pyridyle, quinolinyle, isoquinolyle, imidazolyle, benzimidazolyle, pyrazolyle, oxazolyle, isoxazolyle, benzoxazolyle, thiazolyle, benzothiazolyle, isothiazolyle, pyridazinyle, pyrimidilyle, pyrazinyle, triazinyle, cinnolinyle, phtalazinyle, quinazolinyle. Le groupe hétéroaryle peut être éventuellement substitué par un ou plusieurs groupes hydroxyle, un ou plusieurs groupes alkoxy, un ou plusieurs atomes d'halogène choisis parmi les atomes de fluor, chlore, brome et iode, un ou plusieurs groupes nitro ($-NO_2$), un ou plusieurs groupes nitrile (-CN), un ou plusieurs groupes aryle, un ou plusieurs groupes alkyles, avec les groupes alkyle, alkoxy et aryle tels que définis dans le cadre de la présente invention.

Le terme « alkoxy » signifie un groupe alkyle, alcényle et alcynyle, tels que définis ci-dessus, lié par un atome d'oxygène (-O-alkyle, O-alcényle, O-alcynyle).

Le terme « aryloxy » signifie un groupe aryle tel que défini ci-dessus, lié par un atome d'oxygène (-O-aryle).

Le terme « hétérocycle » désigne de manière générale un substituant mono- ou polycyclique, comportant de 5 à 10 membres, de préférence de 5 à 7 membres, saturé ou instauré, contenant de 1 à 4 hétéroatomes choisis indépendamment l'un de l'autre, parmi l'azote, l'oxygène, le bore, le silicium, le phosphore et le soufre. A titre indicatif, on peut citer les substituants morpholinyle, pipéridinyle, pipérazinyle, pyrrolidinyle, imidazolidinyle, imidazolinyle, pyrazolidinyle, tétrahydrofuranyle, tétrahydropyranyle, thianyle, oxazolidinyle, isoxazolidinyle, thiazolidinyle, isothiazolidinyle. L'hétérocycle peut être éventuellement substitué par un ou plusieurs groupes hydroxyle, un ou plusieurs groupes alkoxy, un ou plusieurs groupes aryle, un ou plusieurs atomes d'halogène choisis parmi les atomes de fluor, chlore, brome et iode, un ou plusieurs groupes nitro ($-NO_2$), un ou plusieurs groupes nitrile (-CN), un ou plusieurs groupes alkyle, avec les groupes alkyle, alkoxy et aryle tels que définis dans le cadre de la présente invention.

Par atome d'halogène, on entend un atome choisi parmi les atomes de fluor, chlore, brome et iode.

Par groupe « silyle » ou « silylé », qui peuvent être utilisé de manière interchangeable, on entend un groupe de formule $[-Si(X)_{3]}$ dans lequel chaque X, indépendamment l'un de l'autre, est choisi parmi un atome d'hydrogène ; un ou plusieurs atomes d'halogène choisis parmi les atomes de fluor, chlore, brome ou iode; un ou plusieurs groupes alkyle ; un ou plusieurs groupes alkoxy ; un ou plusieurs groupes aryle ; un ou plusieurs groupes siloxy ; un ou plusieurs groupes silyle ; avec les groupes alkyle, alkoxy, aryle et siloxy tels que définis dans le cadre de la présente invention.

Par groupe « siloxy », on entend un groupe silylé, tel que défini ci-dessus, lié par un atome d'oxygène ($-O-Si(X)_3$) avec X tel que défini ci-dessus. Au sens de l'invention, par « hétérocycle silylé », on entend un substituant mono- ou polycyclique, comportant de 5 à 15 membres, de préférence de 5 à 7 membres, saturé ou insaturé, contenant au moins un atome de silicium et éventuellement au moins un autre hétéroatome choisi parmi l'azote, l'oxygène et le soufre. Ledit hétérocycle silylé peut être éventuellement substitué par un ou plusieurs groupes hydroxyle ; un ou plusieurs groupes alkyle, un ou plusieurs groupes alkoxy ; un ou plusieurs atomes d'halogène choisis parmi les atomes de fluor, chlore, brome et iode; un ou plusieurs groupes aryle, avec les groupes alkyle, alkoxy et aryle tels que définis dans le cadre de la présente invention. Parmi les hétérocycles silylés, on peut citer par exemple, le 1-silacyclo-3-pentène ou le 1-méthyl-1-hydrido-1-silacyclopentadiène comme représentés ci-dessous :

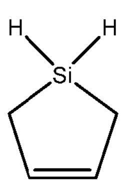

1-silacyclo-3-pentène

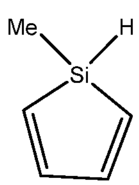

1-méthyl-1-hydrido-1-silacyclopentadiène

Parmi les hétérocycles silylés, on peut encore citer, par exemple, le méthyle siloxane, le 1-phényle-1-silacyclohexane, le 1-sila-bicyclo[2.2.1]héptane, le 1-méthyle-1-silacyclopentane, le 9,9-dihydro-5-silafluorène répondant aux formules semi-développées suivantes :

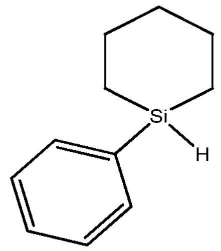

1-phenyl-1-silacyclohexane

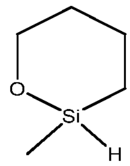

methyl siloxane

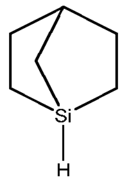

1-sila-bicyclo[2.2.1]heptane

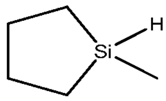

1-methyl-1-silacyclopentane

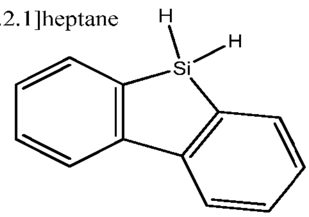

9,9-dihydro-5-silafluorene

Par polyol on entend un composé organique caractérisé par la présence d'un certain nombre de groupements hydroxyle (-OH). Dans le cadre de cette invention un composé polyol contient au moins deux groupements hydroxyle. Plus précisément, on entend par polyol un composé de formule Y-$(OH)_m$, dans lequel m est supérieur ou égale à 1, et Y est choisi parmi un ou plusieurs groupes alkyle, un ou plusieurs groupes alkoxy, un ou plusieurs groupes siloxy, un ou plusieurs groupes aryle, un ou plusieurs groupes hétéroaryle avec les groupes alkyle, alkoxy, siloxy, aryle et hétéroaryle, tels que définis dans le cadre de la présente invention.

Par groupe « amino », on entend un groupe de formule -$NR^4R^5$, dans laquelle :

- $R^4$ et $R^5$ représentent, indépendamment l'un de l'autre, un atome d'hydrogène, un groupe alkyle, un groupe alcényle, un groupe alcynyle, un groupe aryle, un groupe hétéroaryle, un hétérocycle, un groupe silylé, un groupe siloxy, avec les groupes alkyle, alcényle, alcynyle, aryle, hétéroaryle, hétérocycle, silylé, et siloxy, tels que définis dans le cadre de la présente invention ; ou
- $R^4$ et $R^5$, pris ensemble avec l'atome d'azote auquel ils sont liés, forment un hétérocycle éventuellement substitué par un ou plusieurs groupes hydroxyle ; un ou plusieurs groupes alkyle ; un ou plusieurs groupes alkoxy ; un ou plusieurs atomes d'halogène choisis parmi les atomes de fluor, chlore, brome et iode; un ou plusieurs groupes nitro (-$NO_2$) ; un ou plusieurs groupes nitrile (-CN) ; un ou plusieurs groupes aryle ; avec les groupes alkyle, alkoxy et aryle tels que définis dans le cadre de la présente invention.

Dans le cadre de l'invention, la subérine désigne un polymère biosourcé principalement trouvé dans les plantes supérieures. Cette substance organique cireuse est imperméable et se trouve sur les parois cellulosiques de certaines cellules végétales, en particulier celles du liège dont elle constitue le principal constituant. La subérine contient deux domaines : celui des polyaliphatiques et celui des polyaromatiques essentiellement formé de dérivés d'acide hydroxycinnamique. La composition exacte de la subérine varie en fonction de l'espèce.

Le tannin désigne un polymère bio-sourcé contenu dans de nombreux végétaux. Cette substance organique peut être par exemple contenue dans les feuilles (sumac) les écorces et les bois (ex : chêne, acacia) et/ou les racines (badan) des végétaux. Ce composé polyphénolique amorphe est surtout utilisé dans le tannage des peaux pour faire du cuir, la fabrication des encres ou en pharmacologie. Il existe trois grandes catégories de tannins : les tannins hydrolysables, les tannins non hydrolysables ou condensés, et les phlorotannins. Cette définition englobe les pseudo-tannins qui sont des tannins de faible poids moléculaire liés à d'autres composés.

Par tannins hydrolysables, au sens de l'invention, on entend des composés constitués des mélanges de polygalloyles glucoses (ce sont des polymères de molécules formées à partir de dérivés de l'acide gallique et le β-D-glucose comme par exemple dans le cas de l'acide tannique) et/ou de dérivés poly-galloyles de l'acide quinique contenant entre 3 et 12 unités d'acide gallique par molécule. Ces composés contiennent essentiellement des liaisons esters liant les unités aromatiques à un polyol, ce qui facilite son hydrolyse par des acides ou des bases faibles.

Par gallotannins, on entend des tannins hydrolysables dérivés de l'acide gallique, dans lesquels l'acide gallique est lié par des liaisons ester à un polyol central. Dans ces composés, les unités galloyles peuvent de même subir des couplages croisés oxydants (en anglais oxidative cross coupling) ou bien des réactions d'estérification. Il existe plusieurs types de gallotannins répartis essentiellement suivant leur composition chimique comme par exemple : les galloyles glucose (ce sont des molécules formées à partir d'une liaison entre l'acide gallique et le β-D-glucose), les galloyles des acides quiniques, les galloyles des acides shikimiques.

Par ellagitannin, au sens de l'invention, on entend des gallotannins ou des groupements galloyles ayant subi un couplage oxydant C-C. Ce couplage intramoléculaire se réalise pour la plupart des plantes entre les atomes de carbones : C2 et C3 ou bien entre C4 et C6. Ce type de polyphénol forme généralement des macrocycles, alors que ceci n'est pas observable avec les gallotannins.

Par catalyseur, au sens de l'invention, on entend tout composé capable de modifier, notamment en augmentant, la vitesse de la réaction chimique à laquelle il participe, et qui est régénéré à la fin de la réaction. Cette définition englobe à la fois les catalyseurs, c'est-à-dire les composés qui exercent leur activité catalytique sans avoir besoin de subir une quelconque modification ou conversion, et les composés (appelés également pré-catalyseurs) qui sont introduits dans le milieu réactionnel et qui y sont convertis en un catalyseur. Dans le procédé de l'invention, le catalyseur est une base de Lewis.

Il est en particulier nécessaire que le catalyseur soit choisi en tenant compte notamment de son encombrement stérique, de son aptitude à activer le composé hydrosilane et de sa solubilité dans le milieu réactionnel.

Au sens de l'invention, une base de Lewis ou nucléophile est une entité chimique dont un des constituants possède un doublet ou plus d'électrons libres ou non liants sur sa couche de valence. Elles peuvent former des liaisons covalentes coordonnées avec un acide de Lewis.

Dans le procédé de l'invention, la base de Lewis peut être un alcoolate de formule (II), un un composé permettant de libérer un fluorure de formule (III) ou un fluorosilicate de formule (IV).

Selon un mode de réalisation particulier de l'invention, le catalyseur de type base de Lewis est un alcoolate de formule (II)

$$(R^6\text{-}O^-)_w\ M^{w+} \qquad \text{(II)}$$

dans laquelle

- w est 1, 2, 3, 4, et 5 ;
- $R^6$ est un alkyle comprenant 1 à 6 atomes de carbone, un alcényle comprenant 2 à 6 atomes de carbone, un alcynyle comprenant 2 à 6 atomes de carbone ou un aryle monocyclique ou polycyclique typiquement bi- ou tri- cyclique comprenant 6 à 20 atomes de carbone ; et
- M est un métal choisi parmi Li, Na, K, Cs ou Rb Cu, Mg, Zn, Ca, Sr, Ba, Pb, Al, Sb, La, Zr, Si, Ti, Sn, Hf, Ge, V.

Dans ce mode de réalisation, de préférence $R^6$ est un alkyle linéaire ou ramifié comprenant 1 à 6 atomes de carbone, choisi parmi le méthyle, l'éthyle, le propyle, le butyle, le pentyle ou le hexyle et leurs isomères ramifiés.

Dans ce mode de réalisation, M est de préférence Li, Na, K ou Rb.

Dans le catalyseur de formule (II), $R^6$ est un alkyle linéaire ou ramifié comprenant 1 à 6 atomes de carbone, choisi parmi le méthyle, l'éthyle, le propyle, le butyle, le pentyle ou le hexyle et leurs isomères ramifiés et M est Li, Na, K ou Rb.

Toujours dans ce mode de réalisation, l'alcoolate est de préférence choisi parmi $CH_3$-OLi, $CH_3$-ONa, $CH_3$-OK, $CH_3$-ORb, $CH_3CH_2$-OK, $(CH_3\text{-}O)_3Al$, $(PhO)_3Al$, $(iPrO)_3Al$ ou tBu-OK.

Dans ce mode de réalisation particulier, le catalyseur de formule (II), est de préférence l'alcoolate est tBu-OK.

Selon un autre mode de réalisation particulier de l'invention, le catalyseur de type base de Lewis est un composé permettant de libérer un fluorure de formule (III) :

$$Y^{z+} - (F^-)_z \qquad \text{(III)}$$

dans laquelle

- z est 1, 2, 3, 4;
- Y est un ammonium d'alkyle dont l'alkyle comprend 1 à 6 atomes de carbone, un ammonium d'alcényle dont l'alcényle comprend 2 à 6 atomes de carbone, un ammonium d'alcynyle dont l'alcynyle comprend 2 à 6 atomes de carbone ou un aryle comprenant 6 à 10 atomes de carbone ; un ammonium de quinine, ou
- Y est un métal choisi parmi Li, Na, K, Cs, Rb, Cu, Zn, Ca, Ba, Al, Zr, Sn.

Dans ce mode de réalisation, de préférence Y est un ammonium d'alkyle dont l'alkyle comprend 1 à 6 atomes de carbone choisi parmi le méthyle, l'éthyle, le propyle, le butyle, le pentyle ou le hexyle et leurs isomères ramifiés.
De préférence, dans le catalyseur de formule (III), Y est un ammonium d'alkyle dont l'alkyle comprend 1 à 6 atomes de carbone choisi parmi le méthyle, l'éthyle, le propyle, le butyle, le pentyle ou le hexyle et leurs isomères ramifiés.
Dans ce mode de réalisation particulier, le catalyseur de formule (III) est de préférence choisi parmi CsF, TMAF (fluorure de tétraméthylammonium) ou TBAF (fluorure de tétrabutylammonium).
Selon un mode de réalisation particulier de l'invention, le catalyseur de type base de Lewis est un fluorosilicate choisi parmi :

- les hexafluorosilicates $SiF_6^{2-}$ avec un contre-ion alcalins choisi parmi Li, Na, K et Cs ; ou
- les fluorosilicates de formule $(R^7)_3SiF_2^-$ avec un contre-ion ammonium d'alkyle de formule $N(R^{10})_4^+$ou un contre-ion sulfonium de formule $S(R^{11})_3^+$; avec

    - $R^7$ étant un alkyle comprenant de 1 à 6 atomes de carbone choisi parmi le méthyle, l'éthyle, le propyle, le butyle, le pentyle ou le hexyle et leurs isomères ramifiés ; ou un aryle comprenant de 6 à 10 atomes de carbone choisi parmi le phényle, le benzyle ou le naphtyle ;

    - $R^{10}$ étant un atome d'hydrogène ; un méthyle, un éthyle, un propyle, un butyle, et leurs isomères ramifiés.

    - $R^{11}$ étant un atome d'hydrogène ; un méthyle, un éthyle, un propyle, un butyle, et leurs isomères ramifiés ou des amines primaires, secondaires ou tertiaires.

Dans ce mode de réalisation particulier, le fluorosilicate est l'hexafluorosilicate de potassium ($K_2SiF_6$), ou le tétrabutylammonium de difluorotriphénylsilicate $(n\text{-}Bu)_4N^+$ $(Ph)_3SiF_2^-$ aussi appelé TBAT.
Selon un autre mode de réalisation particulier de l'invention, le catalyseur de type base de Lewis est un amidure primaire ou secondaire ou un dérivé guanidine choisi parmi

- les guanidinates bicycliques de sodium ou potassium, notamment le sel de sodium ou de potassium de l'anion du 1,3,4,6,7,8-hexahydro-2H-pyrimido[1,2-a]pyrimidine (ou Hhpp),
- la guanidine,
- la 1,5,7-triazabicyclo[4.4.0]dec-5-ène (ou TBD).

Selon un autre mode de réalisation particulier de l'invention, le catalyseur de type base de Lewis peut aussi être un hétérocycle carbénique de formule générale (IV) :

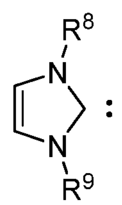

(IV)

dans laquelle

- $R^8$ et $R^9$ représentent, indépendamment l'un de l'autre, un alkyle comprenant 1 à 6 atomes de carbone, un alcényle comprenant 2 à 6 atomes de carbone, un alcynyle comprenant 2 à 6 atomes de carbone ou un aryle bi- ou tricyclique comprenant 6 à 20 atomes de carbone.

Dans ce mode de réalisation, de préférence $R^8$ et $R^9$ indépendamment l'un de l'autre, est un alkyle comprenant de 1 à 6 atomes de carbone choisi parmi le méthyle, l'éthyle, le propyle, le butyle, le pentyle ou le hexyle et leurs isomères ramifiés ; ou un aryle comprenant de 6 à 10 atomes de carbone choisi parmi le phényle, le benzyle, le naphtyle.

L'hétérocycle carbènique peut être choisi,par exemple, parmi le carbène de 2,6-di(1,3-diisopropyl)imidazolium, le carbène de 1,3-bis(1-adamantanyl)imidazolium ou encore le carbène de 1,3-bis-(2,6-diisopropylphenyl)imidazolinium.

Selon un autre mode de réalisation particulier de l'invention, le catalyseur de type base de Lewis peut être un carbonate de formule (V)

$$M'_2CO_3 \qquad (V)$$

dans laquelle

- M' est un métal alcalin choisi parmi Li, Na, K, Cs ou Rb.

Dans ce mode de réalisation, M' est plus particulièrement Na, K ou Cs.

Tous les catalyseurs préférés, qu'ils soient de formule (II), (III), (IV) ou (V), sont commerciaux et peu onéreux comme indiqué ci-dessous. Par exemple,

- tBu-OK est un catalyseur très actif et polyvalent pour la réduction de plastiques oxygénés. Le métal potassium est un métal abondant et peu cher ;
- TBAT est un catalyseur qui ne contient pas de métal, peut être facilement obtenu anhydre et est très actif ; et
- TBAF est un catalyseur sans métal très bon marché. Il peut être obtenu commercialement sous forme de solution 1M dans le THF.

Certaines des abréviations utilisées dans le cadre de l'invention sont représentées ci-dessous :

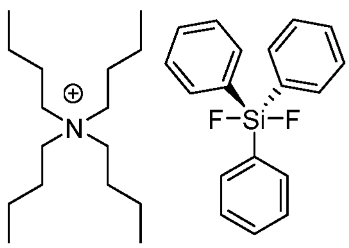

TBAT

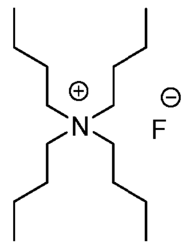

TBAF

Les catalyseurs peuvent, le cas échéant, être immobilisés sur des supports hétérogènes afin d'assurer une séparation facile dudit catalyseur et/ou son recyclage. Lesdits supports hétérogènes peuvent être choisis parmi les supports à base de gel de silice et de polymères plastiques comme, par exemple, le polystyrène ; les supports carbonés choisis parmi les nanotubes de carbone ; le carbure de silice ; l'alumine ; et le chlorure de magnésium ($MgCl_2$).

Selon un mode de réalisation particulier de l'invention, le procédé de dépolymérisation, met en œuvre un composé hydrosilane de formule (I) dans laquelle $R^1$, $R^2$ et $R^3$ représentent, indépendamment l'un de l'autre, un atome d'hydrogène ; un groupe hydroxyle ; un groupe alkyle choisi parmi le méthyle, l'éthyle, le propyle, le butyle, et leurs isomères ramifiés ; un groupe alkoxy dont le radical alkyle est choisi parmi le méthyle, l'éthyle, le propyle, le butyle et leurs isomères ramifiés ; un groupe alkoxy dont le radical alkyle est choisi parmi le méthyle, l'éthyle, le propyle, le butyle et leurs isomères ramifiés ; un groupe aryle choisi parmi le phényle et le benzyle ; un groupe aryloxy dont le radical aryle est choisi parmi le phényle et le benzyle ; un groupe siloxy ($-O-Si(X')_3$) dont chaque X', indépendamment l'un de l'autre, est choisi parmi un atome d'hydrogène, un groupe alkyle choisi parmi le méthyle, l'éthyle, le propyle, un groupe aryle choisi parmi le phényle et le benzyle, un organosilane polymérique de formules générales

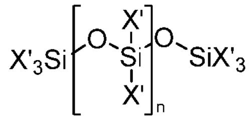

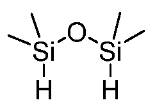

TMDS

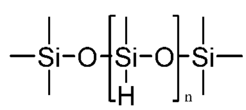

PMHS

dans laquelle X' est tel que défini ci-dessus et n est un nombre entier compris entre 1 et 20000, avantageusement entre 1 et 5000, plus avantageusement entre 1 et 1000 ; lesdits groupes alkyle, alkoxy, aryle, aryloxy, siloxy et aryle étant éventuellement substitués.

Dans un mode de réalisation particulier de l'invention, le procédé de dépolymérisation, met en œuvre un composé hydrosilane de formule (I) dans laquelle $R^1$, $R^2$ et $R^3$ représentent, indépendamment l'un de l'autre, un atome d'hydrogène ; un groupe alkyle choisi parmi le méthyle, l'éthyle, le propyle et son isomère ramifié ; un groupe aryle choisi parmi le benzyle et le phényle ; un groupe siloxy choisi parmi le polydiméthylsiloxane (PDMS), le polyméthylhydroxysiloxane (PMHS) et le tétraméthyldisiloxane (TMDS).

Le PMHS et le TMDS qui sont deux sous-produits de l'industrie du silicone, peuvent être valorisés dans le procédé de dépolymérisation selon l'invention. L'utilisation de deux sous-produits industriels pour créer des molécules ayant une haute valeur ajoutée, est économiquement très avantageuse.

Comme déjà indiqué, le procédé de dépolymérisation se réalise dans des conditions opératoires très douces : température et pression faibles, durée de réaction relativement courte. Une conversion totale du ou des réactifs de départ peut être obtenue en quelques minutes à quelques heures. Il est à noter que la conversion est exprimée par rapport au matériau polymère oxygéné.

Dans le procédé selon l'invention, la dépolymérisation peut être effectuée sous une pression d'un ou d'un mélange de gaz inerte(s) choisi(s) parmi l'azote et l'argon, ou des gaz générés par le procédé notamment du hydrosilane $SiH_4$ et d'hydrogène. La pression peut être comprise entre 0,2 et 5 bars, de préférence entre 1 et 2 bars, bornes incluses.

La dépolymérisation peut être réalisée à une température comprise entre 0 et 100°C, de préférence entre 15 et 75°C, bornes incluses.

La durée de la réaction dépend du taux de conversion du composé hydrosilane de formule (I), de la nature de l'hydrosilane de formule (I) ainsi que de la nature du matériau polymère de départ.

La dépolymérisation peut être effectuée pendant une durée de 1 minute à 200 heures, avantageusement de 1 minute à 48 heures, de préférence de 10 minutes à 48 heures, bornes incluses.

La dépolymérisation, en particulier la réaction entre les différents réactifs, peut avoir lieu dans un ou un mélange d'au moins deux solvant(s) choisi(s) parmi :

- les hydrocarbures aromatiques, de préférence, choisis parmi le benzène, le toluène, le mésitylène ;
- les éthers, de préférence choisis parmi le THF, le diéthyle éther, le Me-THF, l'anisole.

La dépolymérisation, en particulier la réaction entre les différents réactifs, peut avoir lieu en l'absence de solvant. Dans ce cas, l'hydrosilane de formule (I) sert à la fois de réactif et de solvant.

On peut citer l'utilisation du TMDS ainsi que l'utilisation du triméthoxysilane ou encore le triéthoxysilane comme hydrosilane servant de réactifs et de solvants pour la dépolymérisation du PLA ou du PET.

Les hydrosilanes de formule (I) et les catalyseurs utilisés dans l'étape de dépolymérisation sont, en général, des composés commerciaux ou peuvent être préparés par les procédés connus de l'homme du métier.

Le rapport molaire entre le composé hydrosilane de formule (I) et le polymère oxygéné peut être compris entre 0,1 et 20, de préférence entre 0,5 et 10, bornes incluses.

La quantité de catalyseur utilisé dans le procédé de dépolymérisation peut être de 0,001 à 1 équivalent molaire, de préférence de 0,001 à 0,9 équivalent molaire, plus préférentiellement de 0,01 à 0,7 équivalent molaire, encore plus préférentiellement de 0,01 à 0,5 équivalent molaire, bornes incluses, par rapport au nombre de mole initial du polymère oxygéné de départ.

Après la dépolymérisation, les composés résultants sont sous forme silylée. Une simple hydrolyse dans des conditions bien connues de l'homme du métier peut alors conduire aux composés aromatiques ou non aromatiques (aliphatiques) saturés ou insaturés correspondants sous leurs formes non silylées.

Dans le cadre de la présente invention, par hydrolyse on entend un procédé de transformation des composés silylés issus de dépolymérisation du polymère oxygéné, en groupements hydroxyles, par une réaction de désilylation. Cette transformation peut se réaliser dans des conditions acides ou basiques ou bien en présence d'ions fluorures, ces conditions étant bien connues de l'homme du métier. Dans le cadre de la présente invention, le procédé d'hydrolyse est, de préférence, choisi parmi : HCl ou $H_2SO_4$ 2 M dans le THF ; NaOH ou KOH 10 % dans un mélange eau/THF ; NaOH ou KOH 10 % dans le methanol ; $TBAF.3H_2O$ dans le THF ; solution commerciale de TBAF (1M) dans le THF.

Une simple filtration peut permettre de récupérer le catalyseur éventuellement supporté et d'éliminer les éventuels sous-produits.

Les composés issus de la dépolymérisation sont obtenus avec une bonne pureté, c'est-à-dire, une pureté supérieure ou égale à 90 % molaire, de préférence comprise entre 90 et 99,9 % molaire. La pureté molaire peut être déterminée par une analyse spectroscopique ou chromatographique par exemple la RMN du proton (RMN $^1H$) ou la chromatographie en phase gazeuse couplée à la spectrométrie de masse (GC-MS). En effet, dans le procédé de l'invention, les composés formés peuvent être facilement purifiés par les techniques de séparation bien connues de

l'homme du métier et classiquement utilisées dans ce domaine, comme par exemple, la chromatographie sur colonne, la distillation pour les produits volatiles, etc. Les composés obtenus étant en général des petites molécules c'est-à-dire des molécules ayant une masse molaire inférieure à 600 g/mol, leur séparation des produits secondaires éventuellement formés qui sont en général des oligomères avec des liaisons ne pouvant être clivées par le procédé de l'invention, est facile compte tenu des propriétés physico-chimiques très différentes desdits oligomères et des composés obtenus.

Le procédé de l'invention est d'une grande robustesse car il est résistant aux contaminants éventuellement présents dans les matériaux polymères commerciaux (comme les traces d'eaux et les traces de métaux) ainsi qu'aux additifs comme les colorants, ajoutés aux matériaux polymères comme par exemple les plastiques.

Le procédé de l'invention peut être utilisé pour le recyclage de matériaux composites comme les résines contenant du PVC et du PET et qui sont problématiques à recycler. En effet, les liaisons esters du PET sont clivées alors que les liaisons C-C du PVC restent intactes.

Ce procédé peut apporter une solution au stockage des déchets en permettant le recyclage du mélange de déchets tels que le PET et de PLA. En effet, étant donné leur ressemblance au niveau des propriétés physiques et visuelles les plastiques (PET) et les bioplastiques (PLA) sont couramment mélangés. Cependant, leur séparation est très coûteuse et les procédés de recyclage actuels ne permettent pas de recycler les deux polymères en même temps. Il existe donc un vrai problème de recyclage de mélanges de plastiques (PET) et de bio-plastiques (PLA). Le procédé de l'invention permet de recycler un mélange de PET et de PLA soit en clivant le PET sélectivement soit en coupant le PET et le PLA et ce en fonction des conditions opératoires choisies.

Ainsi, l'invention a pour objet l'utilisation du procédé de dépolymérisation de l'invention pour le recyclage de matériaux plastiques contenant au moins un polymère oxygéné au sens de l'invention. En particulier, l'invention a pour objet l'utilisation du procédé de l'invention pour le recyclage des plastiques ou des mélanges de plastiques contenant au moins un polymère oxygéné, c'est-à-dire un polymère ou copolymère dont la chaîne principale comporte des fonctions esters et/ou carbonates, comme par exemple le PLA, le PET, le PC-BPA, etc.

L'invention a encore pour objet un procédé de recyclage de matériaux plastiques ou des mélanges de matériaux plastiques contenant au moins un polymère oxygéné au sens de l'invention, c'est-à-dire un polymère ou copolymère dont la chaîne principale comporte des fonctions esters et/ou carbonates, comme par exemple le PLA, le PET, le PC-BPA, comprenant (i) une étape de dépolymérisation de matériaux polymères oxygénés selon l'invention, éventuellement (ii) une étape d'hydrolyse et éventuellement (iii) une étape de fonctionnalisation et/ou défonctionnalisation. La défonctionnalisation ici signifie la réduction de l'alcool en alcane *i.e.* «remplacer» l'oxygène de la molécule par un hydrogène.

A l'issu du procédé de dépolymérisation de l'invention et après hydrolyse, des composés aromatiques ou non aromatiques (ou aliphatiques) saturés ou insaturés mono- ou polyoxygénés comme, par exemple, di- et /ou tri-oxygénés, de poids moléculaire inférieur à 600 g/mol, comme par exemple, , les coniférols substitués, le phénol, les polyols aromatiques, les quinines, les dérivés de catéchols et d'hydroxycatéchol peuvent être obtenus lorsque le polymère oxygéné de départ contient des motifs aromatiques. Les $\alpha$-$\omega$ diols tels que l'éthylène glycol, le diéthylène glycol, le 1,6-hexanediol le 1,4-butanediol ainsi que le méthanol, le propylène glycol peuvent, par exemple, être obtenus lorsque les polymères contiennent des motifs aliphatiques saturés ou insaturés. Ces composés peuvent être utilisés comme carburant, intermédiaires de synthèse, matières premières dans les secteurs de la construction, dans l'industrie pétrochimique, dans l'industrie électrique, dans l'industrie électronique, dans l'industrie de textile, dans l'industrie aéronautique, dans l'industrie pharmaceutique, dans l'industrie cosmétique, dans l'industrie agrochimique.

L'invention a donc pour objet un procédé de préparation de composés aromatiques mono-, di-, et/ou tri-cycliques dont chaque cycle peut éventuellement être mono-, ou polyoxygénés comme, par exemple, di- et /ou tri-oxygénés di- et /ou tri-oxygénés comprenant (i) une étape de dépolymérisation de polymères oxygénés selon le procédé de l'invention, éventuellement (ii) une étape d'hydrolyse pour former les composés non silylés correspondants, éventuellement (iii) une étape de fonctionnalisation et/ou défonctionnalisation, pour obtenir d'autres composés de hautes valeurs ajoutées.

Dans le cadre de la présente invention, le terme « fonctionnalisation » signifie le remplacement de l'oxygène par une autre fonction. A ce titre on peut citer, par exemple la substitution des oxygènes par des halogènes (*i.e.* chlore, brome, iode), des azotures, des amines, ou l'oxydation des alcools ou encore l'acylation ou la déshydratation des oxygènes.

Dans le cadre de la présente invention, le terme « défonctionnalisation » signifie le remplacement de l'oxygène par un hydrogène en passant, éventuellement, par une étape de fonctionnalisation comme définie ci-dessus.

L'invention a également pour objet un procédé de préparation de composés non aromatiques (ou aliphatiques) saturés ou insaturés mono-, ou polyoxygénés comme, par exemple, di- et /ou tri-oxygénés comprenant (i) une étape de dépolymérisation de polymères oxygénés selon le procédé de l'invention, éventuellement (ii) une étape d'hydrolyse pour former les composés non silylés correspondants et éventuellement (iii) une étape de fonctionnalisation et/ou défonctionnalisation.

Les composés non aromatiques (ou aliphatiques) saturés ou insaturés mono-, ou polyoxygénés comme, par exemple, di- et /ou tri-oxygénés et/ou de composés aromatiques mono-, di-, et/ou tri-cycliques dont chaque cycle peut éventuellement être mono-, ou polyoxygénés comme, par exemple, di- et/ou tri-oxygénés, obtenus par le procédé de

dépolymérisation de matériaux polymères oxygénés selon l'invention, peuvent être utilisés dans la fabrication de combustibles, de composants électroniques, de polymères plastiques, de caoutchouc, de médicaments, de vitamines, de produits cosmétiques, de parfums, de produits alimentaires, de fils et fibres synthétiques, de cuirs synthétiques, de colles, de pesticides, d'engrais.

Ainsi, l'invention a, également, pour objet un procédé de fabrication de combustibles, de composants électroniques, de polymères plastiques, de caoutchouc, de médicaments, de vitamines, de produits cosmétiques, de parfums, de produits alimentaires, de fils et fibres synthétiques, de cuirs synthétiques, de colles, de pesticides, d'engrais comprenant (i) une étape de dépolymérisation de matériaux polymères oxygénés selon le procédé de l'invention, éventuellement (ii) une étape d'hydrolyse pour former, par exemple, les composés non aromatiques (ou aliphatiques) saturés ou insaturés mono-, ou polyoxygénés comme, par exemple, di- et /ou tri-oxygénés et/ou de composés aromatiques mono-, di-, et/ou tri-cycliques dont chaque cycle peut éventuellement être mono-, ou polyoxygénés comme, par exemple, di- et/ou tri-oxygénés, et éventuellement (iii) une étape de fonctionnalisation et/ou défonctionnalisation.

D'autres avantages et caractéristiques de la présente invention apparaîtront à la lecture des exemples ci-dessous donnés à titre illustratif et non limitatif et des figures annexées dans lesquelles :

- La figure 1 représente la réduction de polymères oxygénés avec des catalyseurs acides de Lewis que sont le **BCF** ($B(C_6F_5)_3$) et le complexe d'iridium selon Cantat et al (WO2016/098021, Feghali et al. Chem Sus Chem, 2015, 8, 980 - 984 et Monsigny et al. ACS Sustainable Chem. Eng., 2018, 6, 10481-10488).
- La figure 2 représente l'utilisation de catalyseur de type acide de Lewis pour la réduction de biomasse. (a) La réduction de la cellulose avec le catalyseur $B(C_6F_5)_3$ (Gagné et al., Angew. Chem. Int. Ed. 2014, 53, 1646 -1649) et (b) la réduction de la lignine avec les catalyseurs $B(C_6F_5)_3$.et **[Ir]** selon Cantat. (Cantat et al. WO2016005836A1, Feghali et al. Energy Environ. Sci., 2015, 8, 2734-2743, Monsigny et al. Green Chem., 2018, 20, 1981-1986).
- La figure 3 représente la réduction de liaisons aryl ether dans des modèles de lignine (c'est-à-dire, de liaison carbone oxygène entre deux aromatiques dans une molécule mimant une liaison présente dans un polymère de lignine) avec le terbutanolate de potassium (KOtBu) de manière non catalytique (2-3 équivalents) (Gruber et al., Chem. Sci., 2013, 4, 1640).
- La figure 4 est une représentation schématique comparant la dépolymérisation du PLA par le procédé de dépolymérisation de l'invention (c) aux procédés de l'état de la technique (Cantat et al. WO2016/098021 et Chem Sus Chem, 2015, 8, 980 - 984 (a) ; Monsigny et al. ACS Sustainable Chem. Eng., 2018, 6 , 10481-10488 (b)).

  (a) Cantat et al. WO2016/098021, le mélange de triethylsilane ($Et_3SiH$, 3.3 équivalents) et de PLA (1 équivalent) en présence de **BCF** (5 mol%), comme catalyseur mène à la production de l'alcool silyls correspondant aux monomères du **PLA (PG-Si** pour propylène glycol silylé) mais aussi à une quantité non négligeable de propane.
  (b) Monsigny et al. ACS Sustainable Chem. Eng., 2018, 6, 10481-10488, dans des conditions similaires avec un catalyseur à base d'iridium **[Ir]** mène à un mélange de produits : **PG-Si** est toujours le composé majoritaire mais à la différence de la réaction au **BCF** le sous-produit majoritaire est le propanol silylé et non le propane.
  (c) Enfin, dans le procédé de l'invention, l'utilisation du **TBAF** qui est une base de Lewis, comme catalyseur, ne permet pas le clivage de liaisons simples $\sigma$-C-O tels que les alcools et les éthers. La réaction de dépolymerisation du PLA en présence de triméthoxysilane qui sert ici également de solvant mène à la formation quantitative du seul produit PG-Si.

## EXEMPLES

Dans les exemples ci-après, seuls les polymères les plus couramment utilisés (par exemple : PCL, PET, PC-PBA et PLA) ont été testés. D'autre part, la quantité d'hydrosilane de formule générale (I) nécessaire pour réaliser la dépolymérisation est largement dépendante du type de matériau polymérique employé pour obtenir les alcools silylés ($-OSiR^{12}R^2R^3$). Il est à noter que, par approximation, et afin de calculer le rendement molaire des réactions de dépolymérisation, le matériau de départ est considéré être exclusivement formé du polymère étudié.

Les rendements obtenus sont de l'ordre de 68 à 99 % molaire par rapport au nombre de mole de monomère dans le polymère de départ. Les conversions ont été calculées en se basant sur des analyses spectroscopiques (RMN $^1H$ et RMN $^{13}C$) en utilisant un spectromètre Bruker DPX 200 MHz et par l'ajout d'un étalon interne (le mésitylène ou le diphénylméthane). Les rendements ont été obtenus à l'aide de la chromatographie en phase gazeuse en utilisant comme étalon, le même composé préalablement synthétisé (courbe d'étalonnage externe). Les données de spectrométrie de masse ont été recueillies sur un appareil Shimadzu GCMS-QP2010 Ultra gas chromatograph mass spectrometer équipé avec une colonne capillaire en silice fondue Supelco SLB™-ms (30 m x 0.25 mm x 0.25 $\mu$m). Les analyses qualitatives de gaz ont été exécutés à l'aide de la chromatographie en phase gazeuse sur un appareil Shimadzu GC-2010 équipé d'une colonne capillaire Carboxen™ 1006 PLOT (30 mx 0,53 mm).

**Protocole expérimental général de dépolymérisation**

1. Sous atmosphère inerte d'argon ou d'azote, l'hydrosilane de formule générale (I), le polymère oxygéné et le solvant (si nécessaire) sont mis sous agitation un tube de Schlenk. La concentration en hydrosilane dans le mélange réactionnel varie de 1,0-6,0 $mol.L^{-1}$ (concentration calculée sur la base de la moitié du volume final de solvant introduit).
2. Ensuite, le catalyseur est ajouté au mélange réactionnel (de 1 à 0,001 équivalents molaire calculés par rapport au nombre de moles de matériau polymère initialement ajouté). La solution est agitée et le tube de Schlenk est laissé ouvert afin d'évacuer les gaz produits par la réaction.
3. Après la fin de l'ajout de la solution et l'arrêt du dégagement gazeux, le tube de Schlenk est fermé et est laissé sous agitation. Dans le cas où le matériau de départ est insoluble, la solubilisation se réalise pendant le temps de réaction vu que les produits finaux sont solubles dans les solvants utilisés. Le suivi de réaction est réalisé par RMN $^1H$ et par GC-MS.
4. Une fois la réaction terminée (temps de réaction de 1 minute à 24 heures), le solvant ainsi que les composés volatils sont évaporés à l'aide d'une rampe à vide ($10^{-2}$ mbar). L'huile obtenue est purifiée à l'aide d'une chromatographie sur gel de silice en utilisant un gradient d'élution de 100 : 0 % jusqu'à 0 : 100 % de pentane : $CH_2Cl_2$.
5. Les produits peuvent être hydrolysés en utilisant NaOH (10 %) dans le méthanol, pour fournir le produit hydrolysé correspondant. La réaction d'hydrolyse dure de 1 minute à 16 heures. Le produit final est obtenu après purification sur colonne chromatographique en utilisant un gradient d'élution de 100 : 0 % jusqu'à 0 : 100 % de $CH_2Cl_2$ : AcOEt.

Un ensemble de résultats est présenté ci-dessous, donnant des exemples de dépolymérisation de matériaux polymères oxygénés synthétiques et semisynthétiques.

Les catalyseurs testés sont le TBAT, le TBAF et le KOtBu.

Les hydrosilanes utilisés sont $PhSiH_3$, $(MeO)_3SiH$, $(EtO)_3SiH$ TMDS et PMHS. Les matériaux polymères oxygénés utilisés sont le PLA, le PC-BPA, le PCL, le PET et le PDO. Le PET utilisé est un PET commercial prélevé de bouteilles de d'Evian.

**<u>Exemple 1:</u> Dépolymérisation du PC-BPA avec le triméthoxysilane ($(MeO)_3SiH$) avec KOtBu** :

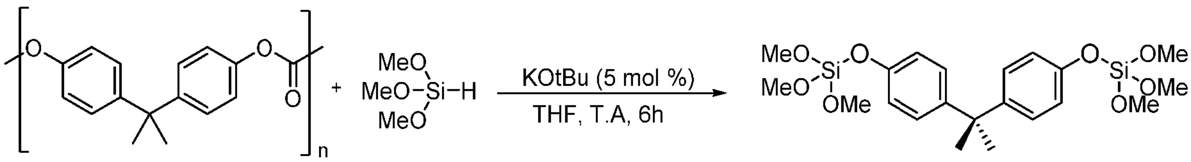


**PC-BPA** 4 équiv. **(IIa 97 %)**

Le PC-BPA commercial (123,2 mg ; 0,5 mmol ; 1 équivalent molaire) et le triméthoxysilane (244 mg ; 2 mmol ; 4 équivalent molaire) ont été ajoutés à 1,5 mL de THF. Le catalyseur KOtBu (0,05 équivalent molaire) est ajouté sous agitation. Après 6h de réaction à température ambiante (20$\pm$5°C), le solvant est évaporé sous vide. Le produit obtenu **IIa** est purifié en utilisant les mêmes conditions que celle décrites dans le mode opératoire général. A l'issu de cette purification, le produit **IIa** est obtenu avec une très grande pureté avec un rendement de 97 % par rapport au matériau de départ introduit.

L'hydrolyse du produit **IIa** en produit dihydroxylé correspondant peut se réaliser directement en ajoutant au mélange réactionnel 10 ml d'une solution de NaOH (10%) dans un mélange méthanol/eau en l'agitant à 25°C pendant 2 h. Le produit hydrolysé (BPA) est obtenu avec un rendement de 88 %, sous forme de solide blanc, après purification sur colonne chromatographique en utilisant les conditions décrites dans le mode opératoire général.

**<u>Exemple 2:</u>dépolymérisation du PC-BPA avec le triméthoxysilane ($(MeO)_3SiH$) avec TBAT**

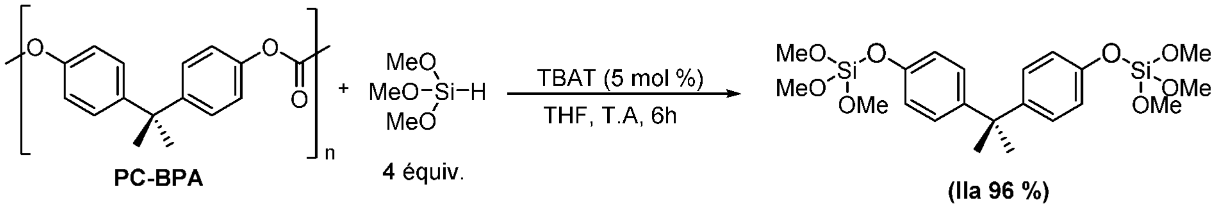


PC-BPA 4 équiv. (IIa 96 %)

Le même mode opératoire employé pour la dépolymérisation du PC-BPA par $(MeO)_3SiH$ avec KOtBu dans l'exemple 1 est employé pour la dépolymérisation avec TBAT (0,05 équivalent molaire). Dans ce cas, 123,2 mg de PC-PBA (0,5 mmol ; 1 équivalent molaire) sont utilisés avec 244 mg de triméthoxysilane (244 mg ; 2 mmol ; 4 équivalent molaire) et 0,05 équivalent molaire de TBAT (13,5 mg, 0,025 mmol, 5 mol%). Après 6 h de réaction la conversion est totale en **IIa**. La purification des produits se réalise en suivant le même mode opératoire décrit dans l'exemple 1.

L'hydrolyse du produit **IIa** conduit à l'obtention de BPA (solide blanc, 92 % de rendement).

**<u>Exemple 3:</u> dépolymérisation du PC-BPA avec le triméthoxysilane ($(MeO)_3SiH$) avec TBAF (1M dans THF)**

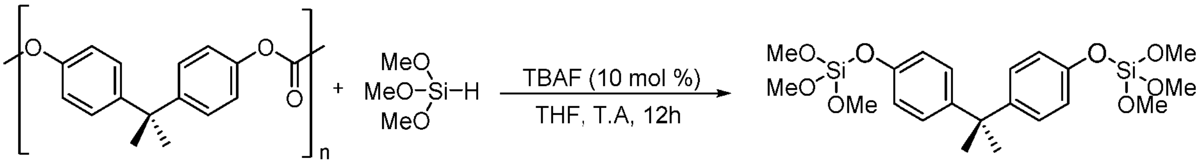


PC-BPA 4 équiv. (IIa 87 %)

Le même mode opératoire employé pour la dépolymérisation du PC-BPA par $(MeO)_3SiH$ avec KOtBu dans l'exemple 1 est employé pour la dépolymérisation avec TBAF (1M dans THF). Dans ce cas, 123,2 mg de PC-PBA (0,5 mmol ; 1 équivalent molaire) sont utilisés avec du triméthoxysilane (244 mg ; 2 mmol ; 4 équivalent molaire) et 50 μL de TBAF (0,05 mmol ; 0,1 équivalent molaire). Après 12 h de réaction la conversion est totale en **IIa**.

La purification des produits se réalise en suivant le même mode opératoire décrit dans l'exemple 1. L'hydrolyse du produit **IIa** conduit à l'obtention de BPA (solide blanc, 92 % de rendement).

**<u>Exemple 4:</u> dépolymérisation du PC-BPA avec le triéthoxysilane ($(EtO)_3SiH$) avec TBAF (1M dans THF)**

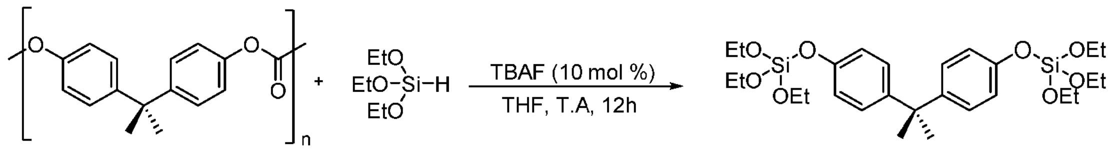


PC-BPA 4 équiv. (IIb 92 %)

Le même mode opératoire employé pour la dépolymérisation du PC-BPA par $(MeO)_3SiH$ avec TBAF dans l'exemple 3 est employé pour la dépolymérisation avec le triethoxysilane. Dans ce cas, 123,2 mg de PC-BPA (0,5 mmol ; 1 équivalent molaire) sont utilisés avec 4équivalent molaire de triéthoxysilane (328 mg ; 2 mmol); et 50 μL de TBAF (0,05 mmol ; 0,1 équivalent molaire). Après 12 h de réaction la conversion est totale en **IIa**. La purification des produits se réalise en suivant le même mode opératoire décrit dans l'exemple 1.

L'hydrolyse du produit **IIb** conduit à l'obtention de BPA (solide blanc, 92 % de rendement).

**<u>Exemple 5:</u> dépolymérisation du PC-BPA avec le TMDS avec TBAF (1M dans THF)**

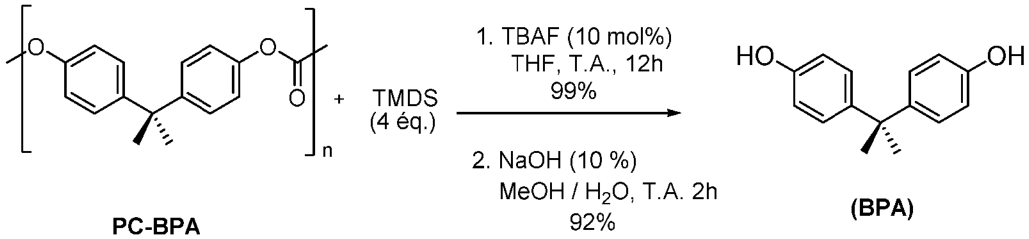


PC-BPA

(BPA)

Le même mode opératoire employé pour la dépolymérisation du PC-BPA par $(MeO)_3SiH$ avec TBAF dans l'exemple 3 est employé pour la dépolymérisation avec TMDS. Dans ce cas, 123,2 mg de PC-PBA (0,5 mmol ; 1 équivalent molaire) sont utilisés avec (266,7 mg ; 2,0 mmol ; 4 équivalent molaire) de TMDS et (50 μL ; 0,05 mmol ; 0,1 équivalent molaire) de TBAF 0,1 équivalent molaire. Après 12 h de réaction la conversion est totale en monomère silylé.

L'hydrolyse des monomères silylés se fait donc directement dans le milieu réactionnel et conduit à l'obtention de BPA (solide blanc, 92 % de rendement).

**<u>Exemple 6:</u> dépolymérisation du PC-BPA avec le PMHS avec TBAF (1M dans THF)**

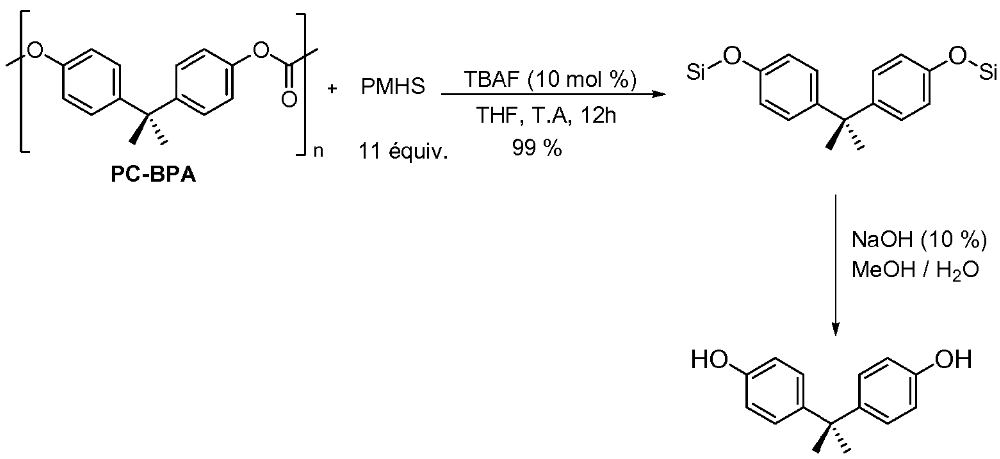


PC-BPA

(BPA 68%)

Le même mode opératoire employé pour la dépolymérisation du PC-BPA par $(MeO)_3SiH$ avec TBAF dans l'exemple 3 est employé pour la dépolymérisation avec PMHS. Dans ce cas, 123,2 mg de PC-PBA (0,5 mmol ; 1 équivalent molaire) sont utilisés avec 330,7 mg de PMHS (5,5 mmol ; 11 équivalent molaire) et 50 μL de TBAF (0,05 mmol ; 0,1 équivalent molaire).

Après 12h, l'hydrolyse de monomères silylés se fait directement dans le milieu réactionnel et conduit à l'obtention de BPA (solide blanc, 68 % de rendement).

**<u>Exemple 7 :</u> dépolymérisation du PET en utilisant TMDS avec TBAF (1M dans THF)**

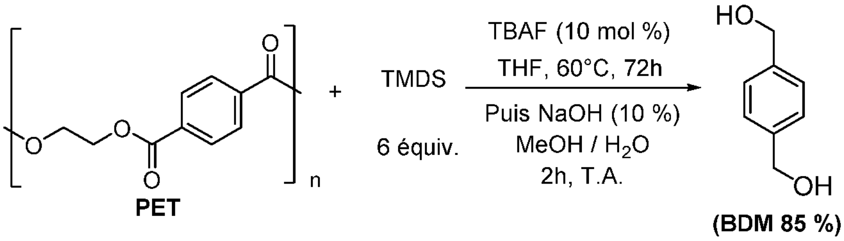


PET

(BDM 85 %)

Le même mode opératoire employé pour la dépolymérisation du PC-BPA par $(MeO)_3SiH$ avec TBAF dans

l'exemple 3 est employé pour la dépolymérisation avec PMHS. Dans ce cas, 96,1 mg de PET (0,5 mmol ; 1 équivalent molaire) sont utilisés avec du TMDS (400,0 mg ; 3,0 mmol ; 6 équivalent molaire) et 0,1 équivalent molaire de TBAF (50 µL ; 0,05 mmol ; 0,1 équivalent molaire). Après 72h à 60°C, l'hydrolyse du produit silylé se fait directement dans le milieu réactionnel et conduit à l'obtention de BDM (solide blanc, 85 % de rendement).

**Exemple 8:dépolymérisation du PCL en utilisant TMDS avec TBAF (1M dans** THF)

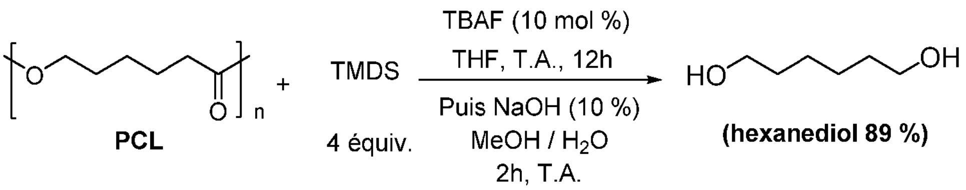


Le même mode opératoire employé pour la dépolymérisation du PC-BPA par $(MeO)_3SiH$ avec TBAF dans l'exemple 3 est employé pour la dépolymérisation avec PMHS. Dans ce cas, (58,2 mg ; 0,5 mmol ; 1 équivalent molaire) de PCL sont utilisés avec 266,7 mg de TMDS (2,0 mmol ; 4 équivalent molaire) et 50 µL de TBAF (0,05 mmol ; 0,1 équivalent molaire). Après 12h à température ambiante (20 + 5°C), l'hydrolyse du produit silylé se fait directement dans le milieu réactionnel et conduit à l'obtention de 1,6-hexanediol (solide blanc, 89 % de rendement).

**Exemple 9: dépolymérisation du PLA en utilisant TMDS avec TBAF (1M dans THF)**

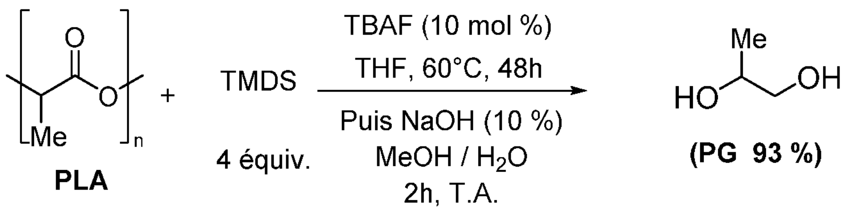


Le même mode opératoire employé pour la dépolymérisation du PC-BPA par $(MeO)_3SiH$ avec TBAF dans l'exemple 3 est employé pour la dépolymérisation avec PMHS. Dans ce cas, (37,0 mg ; 0,5 mmol ; 1 équivalent molaire) de PLA sont utilisés avec 266,7 mg de TMDS (2,0 mmol ; 4 équivalent molaire) et 50 µL de TBAF (0,05 mmol ; 0,1 équivalent molaire). Après 48h à 60°C, l'hydrolyse du produit silylé se fait directement dans le milieu réactionnel et conduit à l'obtention de propylène glycol (huile blanche, 93 % de rendement).

**Exemple 10 : dépolymérisation du PDO en utilisant le TMDS avec le TBAF (1M dans THF)**

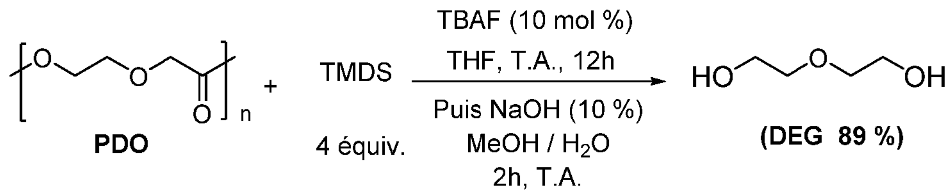


Le même mode opératoire employé pour la dépolymérisation du PC-BPA par $(MeO)_3SiH$ avec TBAF dans l'exemple 3 est employé pour la dépolymérisation avec PMHS. Dans ce cas, 52,2 mg de PDO (0,5 mmol ; 1 équivalent molaire) sont utilisés avec 266,7 mg de TMDS (2,0 mmol ; 4 équivalent molaire) et 50 µL de TBAF (0,05 mmol ; 0,1 équivalent molaire). Après 12h à température ambiante (20 ± 5°C), l'hydrolyse de diéthylèneglycol silylé se fait directement dans le milieu réactionnel et conduit à l'obtention de diéthylèneglycol DEG (huile incolore, 89 % de rendement).

**Exemple 11 :**

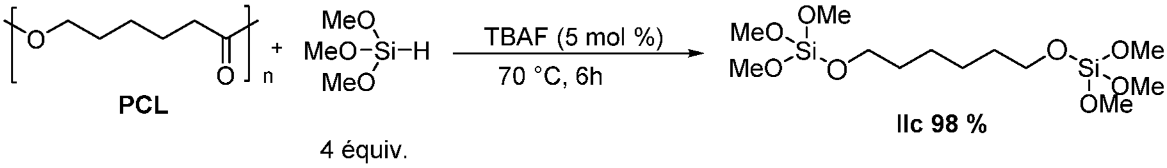


Le même mode opératoire employé pour la dépolymérisation du PC-BPA par $(MeO)_3SiH$ avec TBAF dans l'exemple 3 est employé pour la dépolymérisation avec de PCL avec $(MeO)_3SiH$. Dans ce cas, (52,2 mg ; 0,5 mmol ; 1 équivalent molaire) de PCL sont utilisés avec (244 mg ; 2 mmol ; 4 équivalent molaire) de $(MeO)_3SiH$ et 2,8 mg ; (0,025 mmol ; 5 mol%) de TBAF sans solvant. Après 6h à 70 °C, la purification du produit silylé se réalise en suivant le même mode opératoire décrit dans l'exemple 1 et est obtenu à 98%.

**Caractérisation des molécules obtenues:**

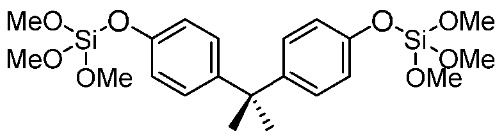

**(IIa 87 %)** **IIa**

**$^1$H NMR** (200 MHz, THF-$d_8$, $Me_4Si$) $\delta$ (ppm) = 7.10 (4H, m, Ar-H), 6.86 (4H, m, Ar-H), 3.58 (18H, s, MeOSi), 1.61 (6H, s, $CH_3$-)

**$^{13}$C NMR** (50 MHz, THF-$d_8$, $Me_4Si$): $\delta$ (ppm) = 151.1, 144.4, 127.5, 118.4, 50.6, 32.7, 30.5.

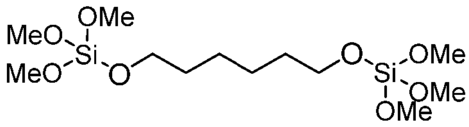

**IIc**

**$^1$H NMR** (200 MHz, THF-$d_8$, $Me_4Si$) $\delta$ (ppm) = 3.73 (4H, t $^3J$ = 6 Hz, O-$CH_2$), 1.55 (4 H, m, O-$CH_2$-$CH_2$-$CH_2$), 1.40 (4 H, m, O-$CH_2$-$CH_2$-$CH_2$-).

**$^{13}$C NMR** (50 MHz, THF-$d_8$, $Me_4Si$): $\delta$ (ppm) = 63.0, 50.2, 32.3, 25.3.

**Les abréviations utilisées sont précisées ci-après :**

PC-BPA = Polcarbonate du bisphénol A
PCL = Poly(caprolactone)
PDO = Poly(dioxanone)
PET = Poly(éthylène térephthalate)
PVC = Poly(chlorure de vinyle)
DEG = Diéthylène glycol
EG= Ethylène glycol
PG= Propylene glycol
BDM= Benzène diméthanol
TPA= acide téréphtalique

PLLA= Poly(L-lactide)
PLA= Acide polylactique
BPA= Bisphénol A
PS= Polystyrène

## Revendications

**1.** Procédé de dépolymérisation de polymères oxygénés par clivage sélectif des liaisons oxygène-carbone des fonctions esters (-CO-O-) et des fonctions carbonates (-O-CO-O-), **caractérisé en ce qu'**il comprend une étape de mise en contact desdits polymères oxygénés avec un composé hydrosilane de formule (I)

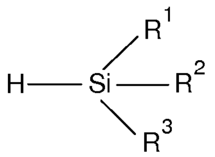

(I)

dans laquelle

- $R^1$, $R^2$ et $R^3$ représentent, indépendamment l'un de l'autre, un atome d'hydrogène, un atome d'halogène, un groupe hydroxyle, un groupe alkyle, un groupe alcényle, un groupe alcynyle, un groupe alkoxy, un groupe aryloxy, un groupe silylé, un groupe siloxy, un groupe aryle, un groupe amino, lesdits groupes alkyle, alcényle, alcynyle, alkoxy, silylé, siloxy, aryle et amino étant éventuellement substitués, ou
- $R^1$ est tel que défini ci-dessus et $R^2$ et $R^3$, pris ensemble avec l'atome de silicium auquel ils sont liés forment un hétérocycle silylé éventuellement substitué ;

en présence d'un catalyseur de type base de Lewis, ledit catalyseur de type base de Lewis étant

- un alcoolate de formule (II)

$$(R^6\text{-}O^-)_w\ M^{w+} \qquad (II)$$

dans laquelle

- w est 1, 2, 3, 4, et 5 ;
- $R^6$ est un alkyle comprenant 1 à 6 atomes de carbone, un alcényle comprenant 2 à 6 atomes de carbone, un alcynyle comprenant 2 à 6 atomes de carbone ou un aryle monocyclique ou polycyclique typiquement bi- ou tri- cyclique comprenant 6 à 20 atomes de carbone ; et
- M est un métal choisi parmi Li, Na, K, Cs ou Rb Cu, Mg, Zn, Ca, Sr, Ba, Pb, Al, Sb, La,. Zr, Si, Ti, Sn, Hf, Ge, V ; ou

- un composé permettant de libérer un fluorure ($F^-$) de formule (III) :

$$Y^{z+} - (F^-)_z \qquad (III)$$

dans laquelle

- z est 1, 2, 3, 4;
- Y est un ammonium d'alkyle dont l'alkyle comprend 1 à 6 atomes de carbone, un ammonium d'alcényle dont l'alcényle comprend 2 à 6 atomes de carbone, un ammonium d'alcynyle dont l'alcynyle comprend 2 à 6 atomes de carbone ou un aryle comprenant 6 à 10 atomes de carbone ; un ammonium de quinine, ou Y est un métal choisi parmi Li, Na, K, Cs, Rb, Cu, Zn, Ca, Ba, Al, Zr, Sn ;

- un fluorosilicate choisi parmi :

• les hexafluorosilicates $SiF_6^{2-}$ avec un contre-ion alcalin choisi parmi Li, Na, K et Cs ; ou

• les fluorosilicates de formule $(R^7)_3SiF_2^-$ avec un contre-ion ammonium d'alkyle de formule $N(R^{10})_4^+$ou un contre-ion sulfonium de formule $S(R^{11})_3^+$; avec

- $R^7$ étant un alkyle comprenant de 1 à 6 atomes de carbone choisi parmi le méthyle, l'éthyle, le propyle, le butyle, le pentyle ou le hexyle et leurs isomères ramifiés ; ou un aryle comprenant de 6 à 10 atomes de carbone choisi parmi le phényle, le benzyle ou le naphtyle ;
- $R^{10}$ étant un atome d'hydrogène ; un méthyle, un éthyle, un propyle, un butyle, et leurs isomères ramifiés.
- $R^{11}$ étant un atome d'hydrogène ; un méthyle, un éthyle, un propyle, un butyle, et leurs isomères ramifiés ou des amines primaires, secondaires ou tertiaires ;

- un amidure primaire ou secondaire un dérivé guanidine choisi parmi

• les guanidinates bicycliques de sodium ou potassium, notamment le sel de sodium ou de potassium de l'anion du 1,3,4,6,7,8-hexahydro-2H-pyrimido[1,2-a]pyrimidine (ou Hhpp),
• la guanidine,
• la 1,5,7-triazabicyclo[4.4.0]dec-5-ène (ou TBD) ;

- un hétérocycle carbènique de formule générale (IV) :

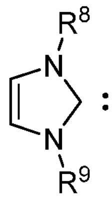

(IV)

dans laquelle

• $R^8$ et $R^9$ représentent, indépendamment l'un de l'autre, un alkyle comprenant 1 à 6 atomes de carbone, un alcényle comprenant 2 à 6 atomes de carbone, un alcynyle comprenant 2 à 6 atomes de carbone ou un aryle bi- ou tri- cyclique comprenant 6 à 20 atomes de carbone ;

- un carbonate de formule (V)

$M'_2CO_3$ (V)

dans laquelle M' est un métal choisi parmi Li, Na, K, Cs ou Rb.

**2.** Procédé selon la revendication 1, **caractérisé en ce que** les polymères oxygénés sont choisis parmi

- les polyesters saturés ou insaturés choisis parmi l'acide polylactique (PLA), le polycaprolactone (PCL), le polyhydroxyalkanoate (PHA), le polyhydroxyvalérate (PHV), le polyéthylène adipate (PEA), le polybutylène succinate (PBS), le poly(3-hydroxybutyrate-co-3-hydroxyvalérate (PHBV), le polyéthylène téréphthalate (PET), le polybutylène téréphthalate (PBT), le polytriméthylène téréphthalate (PTT), le polyéthylène naphthalate (PEN), la polydioxanone (PDO) ;
- les polycarbonates choisis parmi le PC-BPA, le polypropylène carbonate (PPC), le polyéthylène carbonate (PEC), le poly(hexaméthylène carbonate), l'allyl diglycol carbonate (ADC) ou CR-39 ; ou
- les tannins hydrolysables choisis parmi les gallotannins, les ellagitannins, ou la subérine.

**3.** Procédé selon l'une des revendications 1 ou 2, **caractérisé en ce que** les polymères oxygénés sont choisis parmi

- les polyesters choisis parmi le PET, le PCL, le PDO ou le PLA;
- les polycarbonates choisis parmi le PC-BPA ou le PPC.
- les tannins hydrolysables choisi parmi les gallotannins, les ellagitannins, ou la subérine.

**4.** Procédé selon l'une quelconque des revendications 1 à 3, **caractérisé en ce que** catalyseur de type base de Lewis

est un alcoolate de formule (II) dans laquelle $R^6$ est un alkyle linéaire ou ramifié comprenant 1 à 6 atomes de carbone, choisi parmi le méthyle, l'éthyle, le propyle, le butyle, le pentyle ou le hexyle et leurs isomères ramifiés et M est Li, Na, K ou Rb.

**5.** Procédé selon l'une quelconque des revendications 1 à 4, **caractérisé en ce que** le catalyseur de type base de Lewis est un alcoolate de formule (II) choisi parmi $CH_3$-OLi, $CH_3$-ONa, $CH_3$-OK, $CH_3$-ORb, $CH_3CH_2$-OK, $(CH_3\text{-}O)_3Al$, $(PhO)_3Al$, $(iPrO)_3Al$ ou tBu-OK.

**6.** Procédé selon l'une quelconque des revendications 1 à 3, **caractérisé en ce que** le catalyseur de type base de Lewis est un composé de formule (III) dans laquelle Y est un ammonium d'alkyle dont l'alkyle comprend 1 à 6 atomes de carbone choisi parmi le méthyle, l'éthyle, le propyle, le butyle, le pentyle ou le hexyle et leurs isomères ramifiés.

**7.** Procédé selon l'une quelconque des revendications 1 à 3, **caractérisé en ce que** le catalyseur de type base de Lewis est un composé de formule (III) choisi parmi CsF, TMAF (fluorure de tétraméthylammonium) ou TBAF (fluorure de tétrabutylammonium).

**8.** Procédé selon l'une quelconque des revendications 1 à 3, **caractérisé en ce que** le catalyseur de type base de Lewis est un fluorosilicate choisi parmi l'ammonium fluorosilicate ($K_2SiF_6$) ou le tétrabutylammonium de difluorotriphénylsilicate $[(n\text{-}Bu)_4N^+ (Ph)_3SiF_2^-]$ aussi appelé TBAT.

**9.** Procédé selon l'une quelconque des revendications 1 à 3, **caractérisé en ce que** le catalyseur de type base de Lewis est un hétérocycle carbénique de formule (IV) choisi parmi le carbène de 2,6-di(1,3-diisopropyl)imidazolium, le carbène de 1,3-bis(1-adamantanyl)imidazolium ou le carbène de 1,3-bis-(2,6-diisopropylphenyl)imidazolinium.

**10.** Procédé selon l'une quelconque des revendications 1 à 9, **caractérisé en ce que** le composé hydrosilane mis en œuvre est un composé hydrosilane de formule (I) dans laquelle $R^1$ $R^2$ et $R^3$ représentent, indépendamment l'un de l'autre, un atome d'hydrogène ; un groupe hydroxyle ; un groupe alkyle choisi parmi le méthyle, l'éthyle, le propyle, le butyle, et leurs isomères ramifiés ; un groupe alkoxy dont le radical alkyle est choisi parmi le méthyle, l'éthyle, le propyle, le butyle et leurs isomères ramifiés ; un groupe alkoxy dont le radical alkyle est choisi parmi le méthyle, l'éthyle, le propyle, le butyle et leurs isomères ramifiés ; un groupe aryle choisi parmi le phényle et le benzyle ; un groupe aryloxy dont le radical aryle est choisi parmi le phényle et le benzyle ; un groupe siloxy ($-O-Si(X')_3$) dont chaque X, indépendamment l'un de l'autre, est choisi parmi un atome d'hydrogène, un groupe alkyle choisi parmi le méthyle, l'éthyle, le propyle, un groupe aryle choisi parmi le phényle et le benzyle, un organosilane polymérique de formules générales

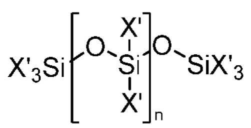

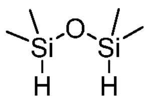

TMDS

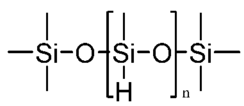

PMHS

dans laquelle X' est tel que défini ci-dessus et n est un nombre entier compris entre 1 et 20000, avantageusement entre 1 et 5000, plus avantageusement entre 1 et 1000 ; lesdits groupes alkyle, alkoxy, aryle, aryloxy, siloxy et aryle étant éventuellement substitués.

**11.** Procédé selon l'une quelconque des revendications 1 à 10, **caractérisé en ce que** le composé hydrosilane mis en œuvre est un composé hydrosilane de formule (I) dans laquelle $R^1$ $R^2$ et $R^3$ représentent, indépendamment l'un de l'autre, un atome d'hydrogène ; un groupe alkyle choisi parmi le méthyle, l'éthyle, le propyle et son isomère ramifié ; un groupe aryle choisi parmi le benzyle et le phényle ; un groupe siloxy choisi parmi le polydiméthylsiloxane (PDMS), le polyméthylhydroxysiloxane (PMHS) et le tétraméthyldisiloxane (TMDS).

**12.** Procédé selon l'une quelconque des revendications 1 à 11, **caractérisé en ce que** le rapport molaire entre le composé hydrosilane de formule (I) et le polymère oxygéné est compris entre 0,1 et 20.

**13.** Procédé selon l'une quelconque des revendications 1 à 12, **caractérisé en ce que** la quantité de catalyseur est de 0,001 à 0,9 équivalent molaire, bornes incluses, par rapport au nombre de mole initial du polymère oxygéné de départ.

**14.** Procédé de recyclage de matériaux plastiques ou des mélanges de matériaux plastiques contenant au moins un polymère oxygéné **caractérisé en ce qu'**il comprend (i) une étape de dépolymérisation de matériaux polymères oxygénés selon l'une quelconque des revendications 1 à 13, éventuellement (ii) une étape d'hydrolyse et éventuellement (iii) une étape de fonctionnalisation et/ou défonctionnalisation.

**15.** Procédé de préparation de composés non aromatiques (ou aliphatiques) saturés ou insaturés mono-, di- et /ou tri-oxygénés, ou de composés aromatiques mono-, di-, et/ou tri-cycliques dont chaque cycle est éventuellement mono-, di- et /ou tri-oxygénés, **caractérisé en ce qu'**il comprend (i) une étape de dépolymérisation de matériaux polymères oxygénés selon l'une quelconque des revendications 1 à 13, éventuellement (ii) une étape d'hydrolyse pour former les composés non silylés correspondants et éventuellement (iii) une étape de fonctionnalisation et/ou défonctionnalisation.

**16.** Procédé de fabrication de combustibles, de composants électroniques, de polymères plastiques, de caoutchouc, de médicaments, de vitamines, de produits cosmétiques, de parfums, de produits alimentaires, de fils et fibres synthétiques, de cuirs synthétiques, de colles, de pesticides, d'engrais, comprenant (i) une étape de dépolymérisation de matériaux polymères oxygénés selon l'une quelconque des revendications 1 à 13, et éventuellement (ii) une étape d'hydrolyse pour former les composés non aromatiques (ou aliphatiques) saturés ou insaturés mono-, di- et /ou tri-oxygénés et/ou de composés aromatiques mono-, di-, et/ou tri-cycliques dont chaque cycle est mono-, di- et/ou tri-oxygénés, éventuellement (iii) une étape de fonctionnalisation et/ou défonctionnalisation.

## Patentansprüche

**1.** Verfahren zur Depolymerisation von mit Sauerstoff angereicherten Polymeren durch selektive Aufspaltung der Sauerstoff-Kohlenstoff-Bindungen der Esterfunktionen (-CO-O-) und der Karbonatfunktionen (-O- CO-O-), **dadurch gekennzeichnet, dass** es einen Schritt des Inkontaktsetzens der mit Sauerstoff angereicherten Polymere mit einer Hydrosilanverbindung der Formel (I) umfasst

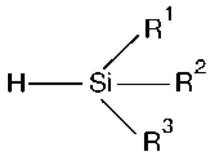

(I)

wobei

- $R^1$, $R^2$ und $R^3$ unabhängig voneinander ein Wasserstoffatom, ein Halogenatom, eine Hydroxylgruppe, eine Alkylgruppe, eine Alkenylgruppe, eine Alkynylgruppe, eine Alkoxygruppe, eine Aryloxygruppe, eine silylhaltige Gruppe, eine Siloxygruppe, eine Arylgruppe, eine Aminogruppe darstellen, wobei die Alkyl-, Alkenyl-, Alkynyl-, Alkoxy-, silylhaltige, Siloxy-, Aryl- und Aminogruppe gegebenenfalls substituiert sind, oder
- $R^1$ wie obenstehend definiert ist und $R^2$ und $R^3$ zusammengenommen mit dem Siliziumatom, an das sie gebunden ist, einen Silylheterozyklus bilden, der gegebenenfalls substituiert ist;

bei Vorhandensein eines Katalysators vom Typ Lewis-Base, wobei der Katalysator vom Typ Lewis-Base

- ein Alkoholat der Formel (II) ist

$$(R^{6-}O^-)_w\ M^{W+} \qquad (II)$$

wobei

- w 1, 2, 3, 4, und 5 ist;
- $R^6$ ein Alkyl mit 1 bis 6 Kohlenstoffatomen, ein Alkenyl mit 2 bis 6 Kohlenstoffatomen, ein Alkynyl mit 2 bis 6 Kohlenstoffatomen oder ein monozyklisches oder polyzyklisches, typischerweise bi- oder trizyklisches,

Aryl mit 6 bis 20 Kohlenstoffatomen ist; und
- M ein Metall ist, ausgewählt aus Li, Na, K, Cs oder Rb Cu, Mg, Zn, Ca, Sr, Ba, Pb, Al, Sb, La, Zr, Si, Ti, Sn, Hf, Ge, V; oder

- eine Verbindung, die Freisetzen eines Fluorids (F) der Formel (III) erlaubt:

$Y^{z+} - (F^-)_z$ (III)

wobei

- z 1, 2, 3, 4 ist;
- Y ein Alkylammonium, dessen Alkyl 1 bis 6 Kohlenstoffatome umfasst, ein Alkenylammonium, dessen Alkenyl 2 bis 6 Kohlenstoffatome umfasst, ein Alkynylammonium, dessen Alkynyl 2 bis 6 Kohlenstoffatome umfasst, oder ein Aryl mit 6 bis 10 Kohlenstoffatomen; ein Chininammonium ist, oder
Y ein Metall ist, ausgewählt aus Li, Na, K, Cs, Rb, Cu, Zn, Ca, Ba, Al, Zr, Sn;

- ein Fluorsilicat, ausgewählt aus:

- den Hexafluorsilicaten $SiF_6^{2-}$ mit einem Alkalin-Gegenion, ausgewählt aus Li, Na, K und Cs; oder
- den Fluorosilicaten der Formel $(R^7)_3SiF_2^-$ mit einem Alkylammonium-Gegenion der Formel $N(R^{10})_{4+}$ oder einem Sulfonium-Gegenion der Formel $S(R^{11})_3^+$; wobei

- $R^7$ ein Alkyl mit 1 bis 6 Kohlenstoffatomen ist, ausgewählt aus Methyl, Ethyl, Propyl, Butyl, Pentyl oder Hexyl und ihren verzweigten Isomeren; oder ein Aryl mit 6 bis 10 Kohlenstoffatomen, ausgewählt aus Phenyl, Benzyl oder Naphtyl;
- $R^{10}$ ein Wasserstoffatom; ein Methyl, ein Ethyl, ein Propyl, ein Butyl, und ihre verzweigten Isomere ist.
- $R^{11}$ ein Wasserstoffatom; ein Methyl, ein Ethyl, ein Propyl, ein Butyl, und ihre verzweigten Isomere oder primäre, sekundäre oder tertiäre Amine ist;

- ein primäres oder sekundäres Amid ein Guanidinderivat, ausgewählt aus

- den bizyklischen Natrium- oder Kaliumguanidinaten, insbesondere Natrium- oder Kaliumsalz des Anions von 1,3,4,6,7,8-Hexahydro-2H- pyrimido[1,2-a]pyrimidin (oder Hhpp),
- Guanidin,
- 1,5,7-Triazabicyclo[4.4.0]dec-5-en (oder TBD) ;

- ein carbenischer Heterozyklus der allgemeinen Formel (IV):

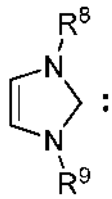

(IV)

wobei

- $R^8$ und $R^9$ unabhängig voneinander ein Alkyl mit 1 bis 6 Kohlenstoffatomen, ein Alkenyl mit 2 bis 6 Kohlenstoffatomen, ein Alkynyl mit 2 bis 6 Kohlenstoffatomen oder ein bi- oder trizyklisches Aryl mit 6 mit 20 Kohlenstoffatomen darstellen;

- ein Karbonat der Formel (V)

$M'_2CO^3$ (V)

wobei M' ein Metall ist, ausgewählt aus Li, Na, K, Cs oder Rb.

2. Verfahren nach Anspruch 1, **dadurch gekennzeichnet, dass** die mit Sauerstoff angereicherten Polymere ausgewählt sind aus

- den gesättigten oder ungesättigten Polyestern, ausgewählt aus Polymilchsäure (PLA), Polycaprolacton (PCL), Polyhydroxyalkanoat (PHA), Polyhydroxyvalerat (PHV), Polyethylenadipat (PEA), Polybutylesuccinat (PBS), Poly(3-hydroxybutyrat-co-3-hydroxyvalerat (PHBV), Polyethylenterephthalat (PET), Polybutylenterephthalat (PBT), Polytrimethylenterephthalat (PTT), Polyethylennaphthalat (PEN), Polydioxanon (PDO);
- den Polycarbonaten, ausgewählt aus PC-BPA, Polypropylencarbonat (PPC), Polyethylencarbonat (PEC), Poly(hexamethylencarbonat), Allyldiglycolcarbonat (ADC) oder CR-39; oder
- den hydrolysierbaren Tanninen, ausgewählt aus den Gallotanninen, den Ellagitanninen, oder Suberin.

3. Verfahren nach einem der Ansprüche 1 oder 2, **dadurch gekennzeichnet, dass** die mit Sauerstoff angereicherten Polymere ausgewählt sind aus

- den Polyestern, ausgewählt aus PET, PCL, PDO oder PLA;
- den Polycarbonaten, ausgewählt aus PC-BPA oder PPC,
- den hydrolysierbaren Tanninen, ausgewählt aus den Gallotanninen, den Ellagitanninen oder Suberin.

4. Verfahren nach einem der Ansprüche 1 bis 3, **dadurch gekennzeichnet, dass** der Katalysator vom Typ Lewis-Base ein Alkoholat der Formel (II) ist, wobei $R^6$ ein lineares oder verzweigtes Alkyl mit 1 bis 6 Kohlenstoffatomen, ausgewählt aus Methyl, Ethyl, Propyl, Butyl, Pentyl oder Hexyl und ihren verzweigten Isomeren ist und M Li, Na, K oder Rb ist.

5. Verfahren nach einem der Ansprüche 1 bis 4, **dadurch gekennzeichnet, dass** der Katalysator vom Typ Lewis-Base ein Alkoholat der Formel (II) ist, ausgewählt aus $CH_3$-OLi, $CH_3$-ONa, $CH_3$-OK, $CH_3$-ORb, $CH_3CH_2$-OK, $(CH_3\text{-}O)_3$A1, $(PhO)_3Al$, $(iPrO)_3$Aloder tBu- OK.

6. Verfahren nach einem der Ansprüche 1 bis 3, **dadurch gekennzeichnet, dass** der Katalysator vom Typ Lewis-Base eine Verbindung der Formel (III) ist, wobei Y ein Alkylammonium ist, dessen Alkyl 1 bis 6 Kohlenstoffatome umfasst, ausgewählt aus Methyl, Ethyl, Propyl, Butyl, Pentyl oder Hexyl und ihren verzweigten Isomeren.

7. Verfahren nach einem der Ansprüche 1 bis 3, **dadurch gekennzeichnet, dass** der Katalysator vom Typ Lewis-Base eine Verbindung der Formel (III) ist, ausgewählt aus CsF, TMAF (Tetramethylammoniumfluorid) oder TBAF (Tetrabutylammoniumfluorid).

8. Verfahren nach einem der Ansprüche 1 bis 3, **dadurch gekennzeichnet, dass** der Katalysator vom Typ Lewis-Base ein Fluorsilicat ist, ausgewählt aus Ammoniumfluorsilicat ($K_2SiF_6$oder Tetrabutylammoniumdifluortriphenylsilicat [$(n\text{-}Bu)_4N^+$ $(Ph)_3SiF_2^-$ ], auch als TBAT bezeichnet.

9. Verfahren nach einem der Ansprüche 1 bis 3, **dadurch gekennzeichnet, dass** der Katalysator vom Typ Lewis-Base ein carbenischer Heterozyklus der Formel (IV) ist, ausgewählt aus 2,6-Di(1,3-diisopropyl)imidazolium-Carben, 1,3-Bis(1-adamantanyl)imidazolium-Carben oder 1,3-Bis-(2,6-diisopropylphenyl)imidazolinium-Carben.

10. Verfahren nach einem der Ansprüche 1 bis 9, **dadurch gekennzeichnet, dass** die eingesetzte Hydrosilanverbindung eine Hydrosilanverbindung der Formel (I) ist, wobei $R^1$, $R^2$ und $R^3$ unabhängig voneinander ein Wasserstoffatom; eine Hydroxylgruppe; eine Alkylgruppe, ausgewählt aus Methyl, Ethyl, Propyl, Butyl, und ihren verzweigten Isomeren; eine Alkoxygruppe, deren Alkylradikal ausgewählt ist aus Methyl, Ethyl, Propyl, Butyl und ihren verzweigten Isomeren; eine Alkoxygruppe, deren Alkylradikal ausgewählt ist aus Methyl, Ehtyl, Propyl, Butyl und ihren verzweigten Isomeren; eine Arylgruppe, ausgewählt aus Phenyl und Benzyl; eine Aryloxygruppe, deren Arylradikal ausgewählt ist aus Phenyl und Benzyl; eine Siloxygruppe ($-O-Si(X')_3$), bei der jedes X unabhängig voneinander ausgewählt ist aus einem Wasserstoffatom, einer Alkylgruppe, ausgewählt aus Methyl, Ethyl, Propyl, einer Arylgruppe, ausgewählt aus Phenyl und Benzyl, einem polymerischen Organosilan der allgemeinen Formeln darstellen

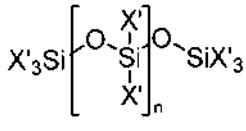

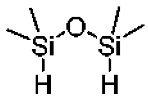

TMDS

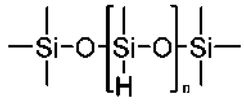

PMHS

wobei X' wie obenstehend definiert ist und n eine ganze Zahl zwischen 1 und 20000, vorteilhafterweise zwischen 1 et 5000, noch vorteilhafterweise zwischen 1 et 1000 ist; wobei die Alkyl-, Alkoxy-, Aryl-, Aryloxy-, Siloxy- und Arylgruppe gegebenenfalls substituiert sind.

**11.** Verfahren nach einem der Ansprüche 1 bis 10, **dadurch gekennzeichnet, dass** die eingesetzte Hydrosilanverbindung eine Hydrosilanverbindung der Formel (I) ist, wobei $R^1$, $R^2$ et $R^3$ unabhängig voneinander ein Wasserstoffatom; eine Alkylgruppe, ausgewählt aus Methyl, Ethyl, Propyl und ihren verzweigten Isomeren; eine Arylgruppe, ausgewählt aus Benzyl und Phenyl; eine Siloxygruppe, ausgewählt aus Polydimethylsiloxan (PDMS), Polymethylhydroxysiloxan (PMHS) und Tetramethyldisiloxan (TMDS) darstellen.

**12.** Verfahren nach einem der Ansprüche 1 bis 11, **dadurch gekennzeichnet, dass** das Molverhältnis zwischen der Hydrosilanverbindung der Formel (I) und dem mit Sauerstoff angereicherten Polymer zwischen 0,1 und 20 beträgt.

**13.** Verfahren nach einem der Ansprüche 1 bis 12, **dadurch gekennzeichnet, dass** die Katalysatormenge 0,001 bis 0,9 Moläquivalente, einschließlich der Grenzwerte, in Bezug auf die anfängliche Anzahl von Molen des ursprünglichen mit Sauerstoff angereicherten Polymers beträgt.

**14.** Verfahren zur Wiederverwertung von Kunststoffmaterialien oder Kunststoffmaterialmischungen, die mindestens ein mit Sauerstoff angereichertes Polymer enthalten, **dadurch gekennzeichnet, dass** es umfasst (i) einen Schritt der Depolymerisation von mit Sauerstoff angereicherten Polymermaterialien nach einem der Ansprüche 1 bis 13, gegebenenfalls (ii) einen Schritt der Hydrolyse und gegebenenfalls (iii) einen Schritt der Funktionalisierung und/oder Defunktionalisierung.

**15.** Verfahren zur Herstellung von gesättigten oder ungesättigten einfach, zweifach und/oder dreifach mit Sauerstoff angereicherten nichtaromatischen (oder aliphatischen) Verbindungen oder von mono-, di- und/oder trizyklischen aromatischen Verbindungen, bei denen jeder Zyklus gegebenenfalls einfach, zweifach und/oder dreifach mit Sauerstoff angereichert ist, **dadurch gekennzeichnet, dass** es umfasst (i) einen Schritt der Depolymerisation von mit Sauerstoff angereicherten Polymermaterialien nach einem der Ansprüche 1 bis 13, gegebenenfalls (ii) einen Schritt der Hydrolyse, um entsprechende nichtsilylhaltige Verbindungen zu bilden, und gegebenenfalls (iii) einen Schritt der Funktionalisierung und/oder Defunktionalisierung.

**16.** Verfahren zur Produktion von Brennstoffen, elektronischen Bauteilen, Kunststoffpolymeren, Kautschuk, Arzneimitteln, Vitaminen, kosmetischen Produkten, Parfüms, Lebensmittelprodukten, künstlichen Garnen und Fasern, künstlichen Ledern, Klebstoffen, Pestiziden, Düngemitteln, umfassend (i) einen Schritt der Depolymerisation von mit Sauerstoff angereicherten Polymermaterialien nach einem der Ansprüche 1 bis 13, (ii) einen Schritt der Hydrolyse zur Bildung von gesättigten oder ungesättigten einfach, zweifach und/oder dreifach mit Sauerstoff angereicherten nichtaromatischen (oder aliphatischen) Verbindungen oder von mono-, di- und/oder trizyklischen aromatischen Verbindungen, bei denen jeder Zyklus einfach, zweifach und/oder dreifach mit Sauerstoff angereichert ist, gegebenenfalls (iii) einen Schritt der Funktionalisierung und/oder Defunktionalisierung.

## Claims

**1.** Method for depolymerizing oxygenated polymers by selective cleavage of the oxygen-carbon bonds of the ester functions (-CO-O-) and of the carbonate functions (-O-CO-O-), **characterized in that** it comprises a step of bringing said oxygenated polymers into contact with a hydrosilane compound of formula (I)

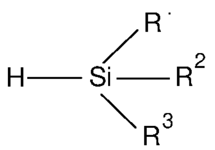

(I)

wherein

- $R^1$, $R^2$ and $R^3$ represent, independently of each other, a hydrogen atom, a halogen atom, a hydroxyl group, an alkyl group, an alkenyl group, an alkynyl group, an alkoxy group, an aryloxy group, a silyl group, a siloxy group, an aryl group, an amino group, said alkyl, alkenyl, alkynyl, alkoxy, silyl, siloxy, aryl and amino groups being optionally substituted, or
- $R^1$ is as defined above and $R^2$ and $R^3$ taken together with the silicon atom to which they are bonded form an optionally substituted silylated heterocycle;

in the presence of a Lewis base catalyst, said Lewis base catalyst being

- an alkoxide of formula (II)

$$(R^6\text{-}O^-)_w\ M^{w+} \qquad \text{(II)}$$

wherein

- w is 1, 2, 3, 4, and 5;
- $R^6$ is an alkyl comprising 1 to 6 carbon atoms, an alkenyl comprising 2 to 6 carbon atoms, an alkynyl comprising 2 to 6 carbon atoms or a monocyclic or polycyclic aryl typically bi- or tri- cyclic comprising 6 to 20 carbon atoms; and
- M is a metal selected from Li, Na, K, Cs or Rb Cu, Mg, Zn, Ca, Sr, Ba, Pb, Al, Sb, La,. Zr, Si, Ti, Sn, Hf, Ge, V; or

- a compound for releasing a fluoride ($F^-$) of formula (III):

$$Y^{z+}\text{-}(F^-)_z \qquad \text{(III)}$$

wherein

- z is 1, 2, 3, 4;
- Y is an alkyl ammonium where the alkyl comprises 1 to 6 carbon atoms, an alkenyl ammonium, where the alkenyl comprises 2 to 6 carbon atoms, an alkynyl ammonium, where the alkynyl comprises 2 to 6 carbon atoms or aryl of 6 to 10 carbon atoms; a quinine ammonium, or
  Y is a metal selected from Li, Na, K, Cs, Rb, Cu, Zn, Ca, Ba, Al, Zr, Sn;

- a fluorosilicate selected from:

  • $SiF_6^{2-}$ hexafluorosilicates with an alkaline counterion selected from Li, Na, K and Cs; or
  • fluorosilicates of formula $(R^7)_3SiF_2^-$ with an alkyl ammonium counterion of formula $N(R^{10})_4^+$ or a sulphonium counterion of formula $S(R^{11})_3^+$; with

    - $R^7$ being an alkyl comprising from 1 to 6 carbon atoms selected from methyl, ethyl, propyl, butyl, pentyl or hexyl and branched isomers thereof; or an aryl comprising from 6 to 10 carbon atoms selected from phenyl, benzyl or naphthyl;
    - $R^{10}$ being a hydrogen atom; a methyl, ethyl, propyl, butyl, and branched isomers thereof.
    - $R^{11}$ being a hydrogen atom; a methyl, ethyl, propyl, butyl, and branched isomers thereof or primary, secondary or tertiary amines;

- a primary or secondary amide a guanidine derivative selected from

  • sodium or potassium bicyclic guanidinates, in particular sodium or potassium salt of the anion of 1,3,4,6,7,8-hexahydro-2H-pyrimido[1,2-a]pyrimidine (or Hhpp),
  • guanidine,
  • 1,5,7-triazabicyclo[4.4.0]dec-5-ene (or TBD);

- a carbene heterocycle of general formula (IV):

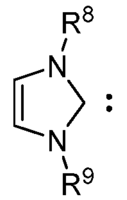

(IV)

wherein

- $R^8$ and $R^9$ represent, independently of each other, an alkyl having 1 to 6 carbon atoms, an alkenyl having 2 to 6 carbon atoms, an alkynyl having 2 to 6 carbon atoms or bi- or tri- cyclic aryl having 6 to 20 carbon atoms;

- a carbonate of formula (V)

$$M'_2CO_3 \quad (V)$$

wherein M' is a metal selected from Li, Na, K, Cs or Rb.

**2.** The method according to claim 1, **characterized in that** the oxygenated polymers are selected from

- saturated or unsaturated polyesters chosen from polylactic acid (PLA), polycaprolactone (PCL), polyhydroxyalkanoate (PHA), polyhydroxyvalerate (PHV), polyethylene adipate (PEA), polybutylene succinate (PBS), poly(3-hydroxybutyrate-*co*-3-hydroxyvalerate (PHBV), polyethylene terephthalate (PET), polybutylene terephthalate (PBT), polytrimethylene terephthalate (PTT), polyethylene naphthalate (PEN), polydioxanone (PDO);
- polycarbonates selected from PC-BPA, polypropylene carbonate (PPC), polyethylene carbonate (PEC), poly(hexamethylene carbonate), allyl diglycol carbonate (ADC) or CR-39; or
- hydrolysable tannins selected from gallotannins, ellagitannins, or suberin.

**3.** The method according to one of claims 1 or 2, **characterized in that** the oxygenated polymers are selected from

- polyesters selected from PET, PCL, PDO or PLA
- polycarbonates selected from PC-BPA or PPC.
- hydrolysable tannins selected from gallotannins, ellagitannins, or suberin.

**4.** The method according to any one of claims 1 to 3, **characterized in that** the Lewis base catalyst is an alkoxide of formula (II) wherein $R^6$ is a linear or branched alkyl comprising 1 to 6 carbon atoms, selected from methyl, ethyl, propyl, butyl, pentyl or hexyl and branched isomers thereof and M is Li, Na, K or Rb.

**5.** The method according to any one of claims 1 to 4, **characterized in that** the Lewis base catalyst is an alkoxide of formula (II) selected from $CH_3$-OLi, $CH_3$-ONa, $CH_3$-OK, $CH_3$-ORb, $CH_3CH_2$-OK, $(CH_3\text{-}O)_3Al$, $(PhO)_3Al$, $(iPrO)_3Al$ or tBu-OK.

**6.** The method according to any one of claims 1 to 3, **characterized in that** the Lewis base catalyst is a compound of formula (III) wherein Y is an alkyl ammonium, the alkyl of which comprises 1 to 6 carbon atoms selected from methyl, ethyl, propyl, butyl, pentyl or hexyl and branched isomers thereof.

**7.** The method according to any one of claims 1 to 3, **characterized in that** the Lewis base catalyst is a compound of formula (III) selected from CsF, TMAF (tetramethylammonium fluoride) or TBAF (tetrabutylammonium fluoride).

**8.** The method according to any one of claims 1 to 3, **characterized in that** the Lewis base catalyst is a fluorosilicate selected from ammonium fluorosilicate ($K_2SiF_6$) or difluorotriphenylsilicate tetrabutylammonium $[(n\text{-}Bu)_4N+ (Ph)_3SiF_2\text{-}]$ also called TBAT.

**9.** The method according to any one of claims 1 to 3, **characterized in that** the Lewis base catalyst is a carbene heterocycle of formula (IV) selected from 2,6-di(1,3-diisopropyl)imidazolium carbene, 1,3-bis(1-adamantanyl)imidazolium carbene or 1,3-bis-(2,6-diisopropylphenyl)imidazolinium carbene.

**10.** The method according to any one of claims 1 to 9, **characterized in that** the hydrosilane compound used is a hydrosilane compound of formula (I) in which $R^1$, $R^2$ and $R^3$ represent, independently of each other, a hydrogen atom; a hydroxyl group; an alkyl group chosen from methyl, ethyl, propyl and butyl, and branched isomers thereof; an alkoxy group, the alkyl radical of which is chosen from methyl, ethyl, propyl, butyl, and branched isomers thereof; an alkoxy group, the alkyl radical of which is chosen from methyl, ethyl, propyl, butyl and branched isomers thereof; an aryl group chosen from phenyl and benzyl; an aryloxy group, the aryl radical of which is chosen from phenyl and benzyl; a siloxy group (-O-Si(X')$_3$) where each X, independently of each other, is selected from a hydrogen atom, an alkyl group selected from methyl, ethyl, propyl, an aryl group selected from phenyl and benzyl, a polymeric organosilane of general formulae

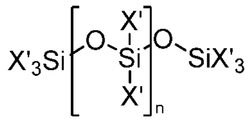

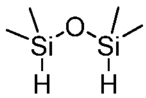

TMDS

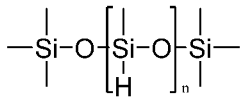

PMHS

wherein X' is as defined above and n is an integer between 1 and 20,000, advantageously between 1 and 5,000, more advantageously between 1 and 1,000; said alkyl, alkoxy, aryl, aryloxy, siloxy and aryl groups being optionally substituted.

**11.** The method according to any one of claims 1 to 10, **characterized in that** the hydrosilane compound used is a hydrosilane compound of formula (I) in which $R^1$, $R^2$ and $R^3$ represent, independently of each other, a hydrogen atom; an alkyl group chosen from methyl, ethyl, propyl and branched isomer thereof; an aryl group chosen from benzyl and phenyl; a siloxy group chosen from polydimethylsiloxane (PDMS), polymethylhydroxysiloxane (PMHS) and tetramethyldisiloxane (TMDS).

**12.** The method according to any one of claims 1 to 11, **characterized in that** the molar ratio between the hydrosilane compound of formula (I) and the oxygenated polymer is between 0.1 and 20.

**13.** The method according to any one of claims 1 to 12, **characterized in that** the amount of catalyst is from 0.001 to 0.9 molar equivalent, including bounds, with respect to the initial mole number of the starting oxygenated polymer.

**14.** A method for recycling plastic materials or mixtures of plastic materials containing at least one oxygenated polymer, **characterized in that** it comprises (i) a step of depolymerizing oxygenated polymer materials according to any one of claims 1 to 13, optionally (ii) a hydrolysing step and optionally (iii) a functionalizing and/or defunctionalizing step.

**15.** A method for preparing saturated or unsaturated mono-, di- and/or tri-oxygenated non-aromatic (or aliphatic) compounds, or of mono-, di- and/or tri-cyclic aromatic compounds, each cycle of which is optionally mono-, di- and/or tri-oxygenated **characterized in that** it comprises (i) a step of depolymerizing oxygenated polymeric materials according to any one of claims 1 to 13, optionally (ii) a hydrolysing step to form the corresponding non-silylated compounds and optionally (iii) a functionalizing and/or defunctionalizing step.

**16.** A method for manufacturing fuels, electronic components, plastic polymers, rubber, drugs, vitamins, cosmetics, perfumes, food products, synthetic yarns and fibres, synthetic leathers, glues, pesticides, fertilizers, comprising (i) a step of depolymerizing oxygenated polymeric materials according to any one of claims 1 to 13, and optionally (ii) a hydrolysing step to form the saturated or unsaturated mono-, di- and/or tri-oxygenated non-aromatic (or aliphatic) compounds and/or mono-, di-, and/or tri-cyclic aromatic compounds each cycle of which is mono-, di- and/or tri-oxygenated, optionally (iii) a functionalizing and/or defunctionalizing step.

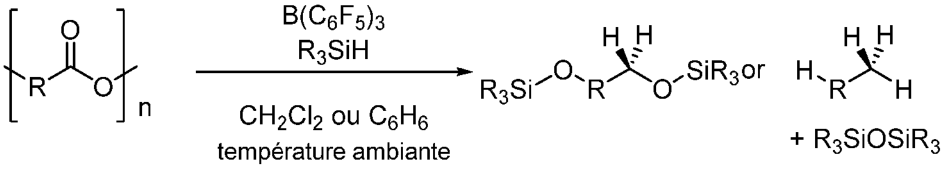


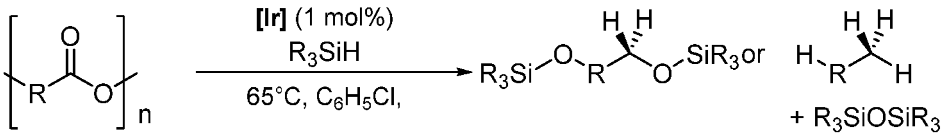


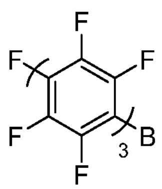

**BCF**

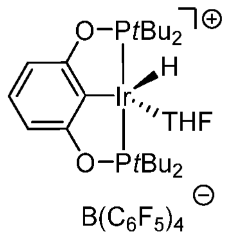

**[Ir]**

**Figure 1**

Gagné *et al.*

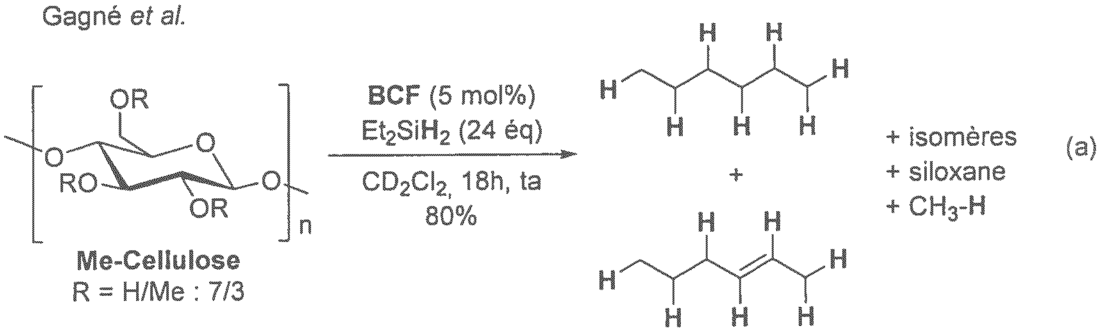


Cantat *et al.*

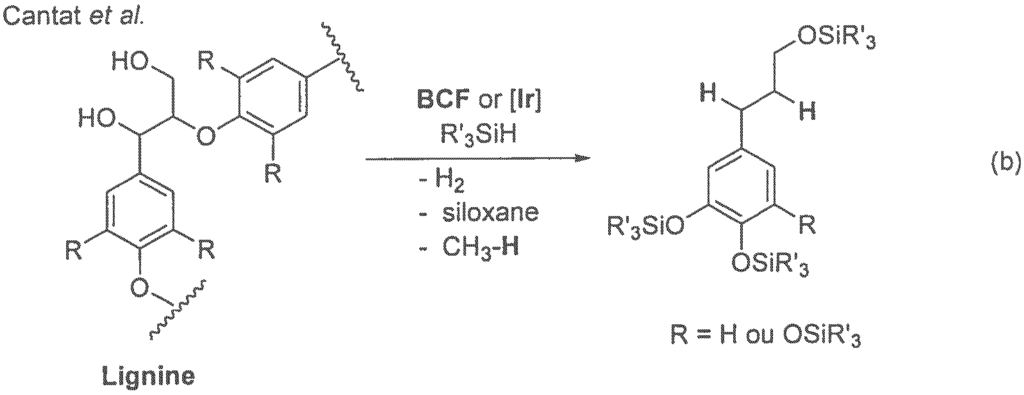


**Figure 2**

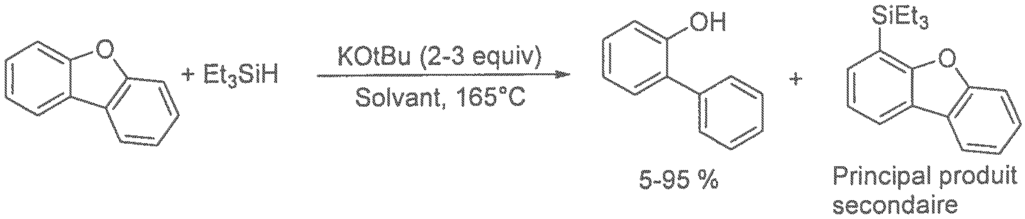


**Figure 3**

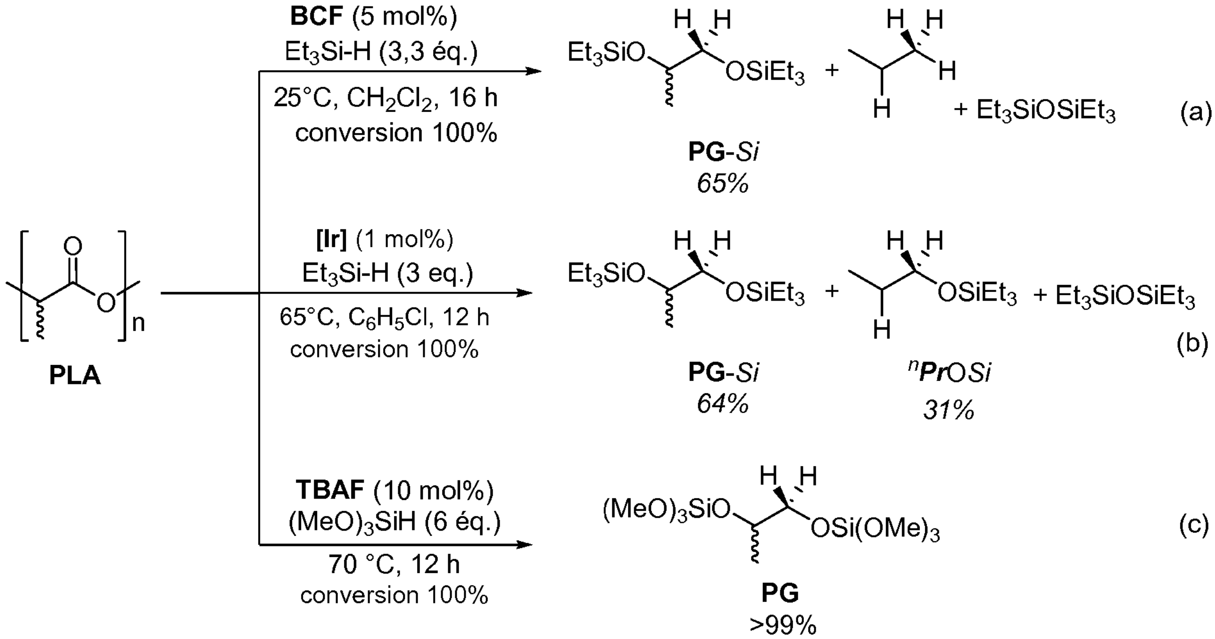


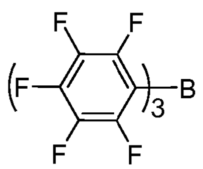

**BCF**

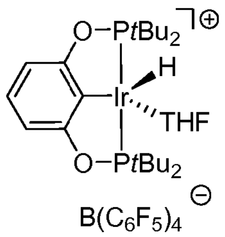

**[Ir]**

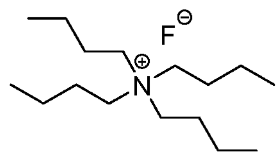

**TBAF**

**Figure 4**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2016098021 A
- WO 2016005836 A1, Cantat


### Littérature non-brevet citée dans la description


- **S.M. AL-SALEM ; P. LETTIERI ; J. BAEYENS.** *Progress in Energy and Combustion Science,* 2010, vol. 36, 103-129
- **S. H. PARK ; S. H. KIM.** *Fashion and Textiles,* 2014, vol. 1, 1-17
- **D. S. ACHILIAS ; D. A. LOUKA ; G. TSINTZOU ; I. A. KOUTSIDIS ; I. TSAGKALIAS ; L. ANDRIOTIS ; N. P. NIANIAS ; P. SIAFAKA.** Recent Advances in the Chemical Recycling of Polymers (PP, PS, LDPE, HDPE, PVC, PC, Nylon, PMMA). INTECH Open Access Publisher, 2012
- **MIAO HONG ; EUGENE Y.-X.** Chemically recyclable polymers: a circular economy approach to sustainability. *Chen, Green Chem.,* 2017, vol. 19, 3692
- **ROBERTSON et al.** *Chem. Commun,* 2014, vol. 50, 4884-4887
- **S. WESTHUES ; J. IDEL ; J. KLANKERMAYER.** Molecular catalyst systems as key enablers for tailored polyesters and polycarbonate recycling concepts. *Sci. Adv.,* 2018, vol. 4, eaat9669
- *Chem Sus Chem,* 2015, vol. 8, 980-984
- *ACS Sustainable Chem. Eng,* 2018, vol. 6, 10481-10488
- **GAGNÉ et al.** Metal-Free Deoxygenation of Carbohydrates. *Angew. Chem. Int. Ed.,* 2014, vol. 53, 1646-1649
- **FEGHALI et al.** *Energy Environ. Sci.,* 2015, vol. 8, 2734-2743
- **MONSIGNY et al.** *Green Chem.,* 2018, vol. 20, 1981-1986
- **K. REVUNOVA ; G. I. NIKONOV.** *Dalton Trans.,* 2014
- *Angew. Chem. Int. Ed.,* 2015, vol. 54, 6931-6934
- **M. DENEUX ; I.C. AKHREM ; D.V. AVETISSIAN ; E.I. MYSOFF ; M.E. VOLPIN.** *Bull. Soc. Chim. France,* 1973, 2638
- **BOYER, J. ; CORRIU, R. J. P. ; PERZ, R. ; REYE, C.** *J. Organomet. Chem.,* 1979, vol. 172, 143
- **CORRIU, R. J. P. ; PERZ, R. ; REYE, C.** *Tetrahedron,* 1983, vol. 39, 999
- **M. FUJITA ; T. HIYAMA.** *J. Am. Chem. Soc,* 1984, vol. 106, 4629
- **DUB, P. A. ; IKARIYA, T.** Catalytic Reductive Transformations of Carboxylic and Carbonic Acid Derivatives Using Molecular Hydrogen. *ACS Catal.,* 2012, vol. 2 (8), 1718-1741
- **GRUBBS et al.** *Chem. Sci.,* 2013, vol. 4, 1640-1645
- **FEGHALI et al.** *Chem Sus Chem,* 2015, vol. 8, 980-984
- **MONSIGNY et al.** *ACS Sustainable Chem. Eng,* 2018, vol. 6, 10481-10488
- **GAGNÉ et al.** *Angew. Chem. Int. Ed.,* 2014, vol. 53, 1646-1649
- **GRUBER et al.** *Chem. Sci.,* 2013, vol. 4, 1640
- **MONSIGNY et al.** *ACS Sustainable Chem. Eng.,* 2018, vol. 6, 10481-10488